# EUROPEAN PATENT SPECIFICATION

(11) **EP 1 361 895 B1**
(45) Date of publication and mention of the grant of the patent: **14.11.2007**
(21) Application number: 02709573.6
(22) Date of filing: 19.02.2002
(51) Int. Cl.: A61K 47/30, A61K 39/385, A61K 38/54, C07D 491/22

(54) **TERMINALLY-BRANCHED POLYMERIC LINKERS AND POLYMERIC CONJUGATES CONTAINING THE SAME**
ENDGRUPPENVERZWEIGTE POLYMERE VERBINDUNGEN UND DIESE ENTHALTENDE POLYMERE KONJUGATE
LIEURS POLYMERIQUES A RAMIFICATION TERMINALE ET CONJUGUES POLYMERIQUES CONTENANT CEUX-CI

(30) Priority: 20.02.2001 US 272511 P
(43) Date of publication of application: 19.11.2003
(73) Proprietor: ENZON, INC., Piscataway, NJ 08854 (US)
(72) Inventor: CHOE, Yun, Hwang, Green Brook, NJ 08812 (US); GREENWALD, Richard, B., Somerset, NJ 08873 (US)
(74) Representative: Prins, Adrianus Willem
(86) International application number: PCT/US2002/004780
(87) International publication number: WO 2002/066066

(56) References cited:
- WO-A-93/24476
- WO-A-96/23794
- WO-A-98/07713
- US-A- 5 965 566
- US-A- 6 011 042
- DATABASE HCAPLUS [Online] GREENWALD ET AL.: 'Drug delivery systems based on trimethyl lock lactonization: poly(ethylene glycol) prodrugs of amino-containing compounds', XP002950994 Retrieved from ACS Database accession no. 2000:44098
- DATABASE HCAPLUS [Online] GREENWALD ET AL.: 'Trialkyl-lock-facilitated polymeric prodrugs of amino-containing bioactive agents', XP002950993 Retrieved from ACS Database accession no. 1999:468433

## Description

### TECHNICAL FIELD

The present invention relates to new types of terminally-activated polymeric materials which are useful in forming long-acting conjugates of bioactive materials. In particular, the invention relates to polymeric-based conjugates having increased therapeutic payloads and methods of preparing the same.

### BACKGROUND OF THE INVENTION

Over the years, several methods of administering biologically-effective materials to mammals have been proposed. Many medicinal agents are available as water-soluble salts and can be included in pharmaceutical formulations relatively easily. Problems arise when the desired medicinal agent is either insoluble in aqueous fluids or is rapidly degraded in vivo. Alkaloids are often especially difficult to solubilize.

One way to solubilize medicinal agents is to include them as part of a soluble prodrug. Prodrugs include chemical derivatives of a biologically-active parent compound which, upon administration, eventually liberate the parent compound in vivo. Prodrugs allow the artisan to modify the onset and/or duration of action of an agent in vivo and can modify the transportation, distribution or solubility of a drug in the body. Furthermore, prodrug formulations often reduce the toxicity and/or otherwise overcome difficulties encountered when administering pharmaceutical preparations. Typical examples of prodrugs include organic phosphates or esters of alcohols or thioalcohols. See Remington's Pharmaceutical Sciences, 16th Ed., A. Osol, Ed. (1980), the disclosure of which is incorporated by reference herein.

Prodrugs are often biologically inert or substantially inactive forms of the parent or active compound. The rate of release of the active drug, i.e. the rate of hydrolysis, is influenced by several factors but especially by the type of bond joining the parent drug to the modifier. Care must be taken to avoid preparing prodrugs which are eliminated through the kidney or reticular endothelial system, etc. before a sufficient amount of hydrolysis of the parent compound occurs.

Incorporating a polymer as part of a prodrug system has been suggested to increase the circulating life of a drug. However, it has been determined that when only one or two polymers of less than about 10,000 daltons each are conjugated to certain biologically active substances such as alkaloid compounds, the resulting conjugates are rapidly eliminated in vivo, especially if a somewhat hydrolysis-resistant linkage is used. In fact, such conjugates are so rapidly cleared from the body that even if a hydrolysis-prone ester linkage is used, not enough of the parent molecule is regenerated in vivo to be therapeutic.

Camptothecin and related biologically active analogs are often poorly water soluble and are examples of substances which would benefit from PEG prodrug technology. A brief overview of some previous work in the field is presented below.

Ohya, et al., J. Bioactive and Compatible Polymers Vol. 10 Jan., 1995, 51-66, disclose doxorubicin-PEG conjugates which are prepared by linking the two substituents via various linkages including esters. The molecular weight of the PEG used, however, is only about 5,000 at most. Thus, the in vivo benefits are not fully realized because the conjugates are substantially excreted prior to sufficient linkage hydrolysis.

U.S. Patent No. 4,943,579 discloses certain simple 20(S)-camptothecin amino acid esters in their salt forms as water soluble prodrugs. The reference does not, however, disclose using an amino acid as part of a linkage which would attach the alkaloid to a relatively high molecular weight polymer in order to form a prodrug. As evidenced by the data provided in Table 2 of the '579 patent, hydrolysis is rapid. Consequently, at physiologic pH, the insoluble base is rapidly generated after injection, binds to proteins and is quickly eliminated from the body before a therapeutic effect can be achieved. A related effort was directed to developing a water-soluble camptothecin sodium salt. Unfortunately, the water-soluble sodium salt of camptothecin remained too toxic for clinical application (Gottlieb et al,. 1970 Cancer Chemother, Rep. 54, 461; Moertel et al,. 1972 ibid, 56, 95; Gottlieb et al., 1972 ibid, 56, 103).

Commonly-assigned PCT publication WO96/23794 describes bis-conjugates in which one equivalent of the hydroxyl-containing drug is attached to each terminal of the polymer. In spite of this advance, techniques which would further increase the payload of the polymer have been sought.

US-A 6 011 042 discloses polymeric prodrugs of aromatic hydroxyl-containing compounds having an ester linkage between the aromatic compound and the polymer. Examples of aromatic hydroxyl-containing compounds are drugs such as hydroxycamptothecins, etoposide, tetracyclines etc.

US-A 5 965 566 discloses prodrug compositions containing hydrolyzable linkages, preferably ester linkages, between a polymer portion and a biologically active moiety or drug, such as paclitaxel, taxotere, camptothecin and podophyllotoxin.

WO 98/07713 discloses water soluble prodrugs having a biologically active moiety attached to a water soluble polymer through a hydrolyzable linkage.

WO 93/24476 discloses a taxol derivative having taxol covalently linked to a water soluble polymeric carrier. The carrier can be branched or unbranched or multi-arm star polyethylene glycol (PEG).

Greenwald et al., "Drug delivery systems based on trimethyl lock lactonization: poly(ethylene glycol) prodrugs of amino-containing compounds", 2000, and "Trialkyl-lock-facilitated polymeric prodrugs of amino-containing bioactive agents", 1999, disclose water soluble double prodrugs of amino-containing drug compounds having reversible linkages between a linear polymer, such as PEG, and the drug compound. The linkage can be cleaved stepwise to first cleave the polymeric portion and then, in vivo, as a result of a tri-alkyl lock type lactonization-type reaction, the drug compound.

Thus, there continues to be a need to provide additional technologies for forming prodrugs of therapeutic moieties such as camptothecin and related analogs. The present invention addresses this need.

### SUMMARY OF THE INVENTION

In one aspect of the invention, compounds of Formula (I) are provided: 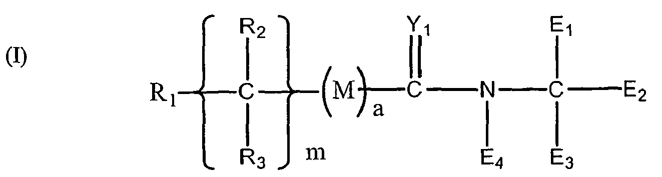 wherein:
R₁ is a polymeric residue;
Y₁ is O, S or NR₄;
M is O, S or NR₅;
(m) is zero or a positive integer, preferably 1 or 2;
(a) is zero or one;
E₁ is 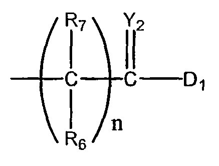
E₂₋₄ are independently H, E₁ or 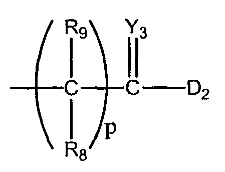
(n) and (p) are independently 0 or a positive integer;
Y₂₋₃ are independently O, S or NR₁₀;
R₂₋₁₀ are independently selected from the group consisting of hydrogen, C₁₋₆ alkyls, C₃₋₁₂ branched alkyl, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
D₁ and D₂ are independently OH, 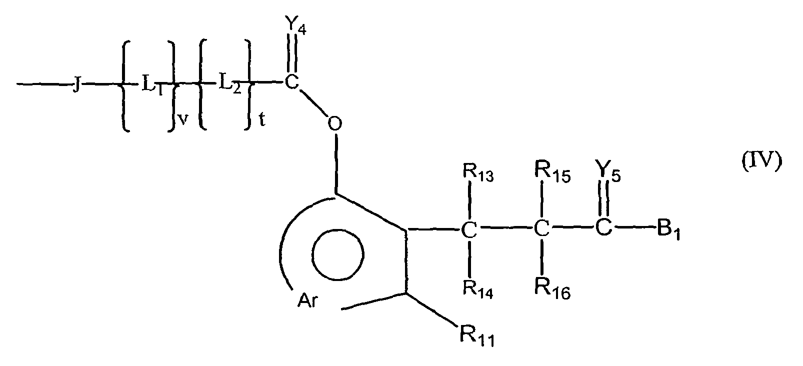 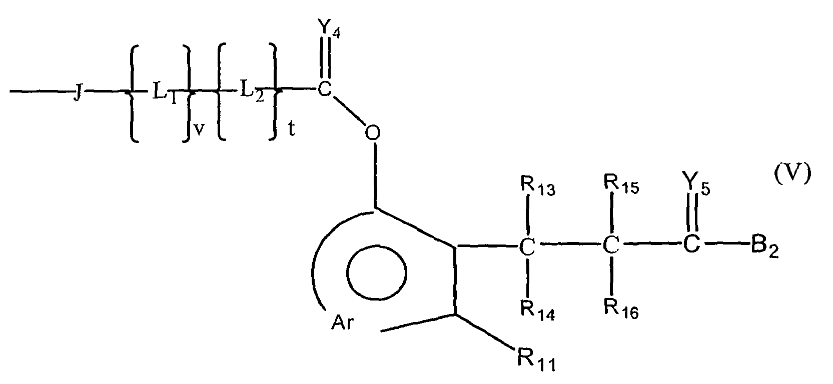
or additional branching groups described below.

Within formulae (IV) and (V), (v) and (t) are independently 0 or a positive integer up to about 6 and preferably about1;
J is NR₁₂ or 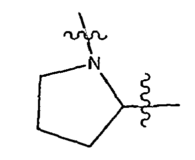 L₁ and L₂ are independently selected bifunctional linkers;
Y₄₋₅ are independently selected from the group consisting of O, S and NR₁₇;
R₁₁₋₁₇ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
Ar is a moiety which when included in Formula (I) forms a multi-substituted aromatic hydrocarbon or a multi-substituted heterocyclic group; and
B₁ and B₂ are independently selected from the group consisting of leaving groups, OH, residues of hydroxyl- or amine-containing moieties.

In one particularly preferred aspect of the invention, the polymeric residue is also substituted on the distal portion with a moiety of formula (II) below: 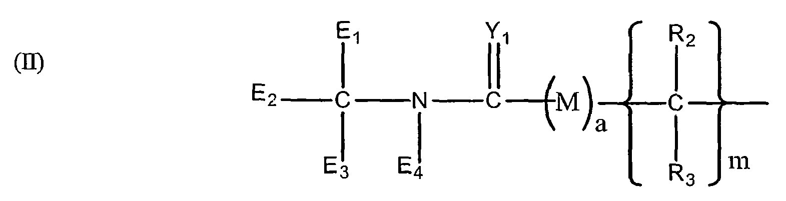 where all variables are as previously defined. Bifunctional compounds are thus formed when the polymeric residue (R₁) includes both an alpha and an omega terminal linking group so that two, four or more equivalents of a biologically active agent, drug or protein, designated herein as B₁ or B₂ can be delivered. An example of such a bifunctional polymer transport form is illustrated below as formula (III): 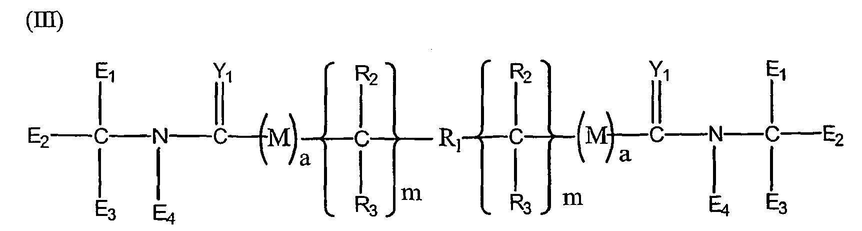 wherein all variables are as described above.

For purposes of the present invention, the term "residue" shall be understood to mean that portion of a biologically active compound which remains after the biologically active compound has undergone a substitution reaction in which the prodrug carrier portion has been attached.

For purposes of the present invention, the term "alkyl" shall be understood to include straight, branched, substituted, e.g. halo-, alkoxy-, and nitro-, C₁₋₁₂ alkyl, C₃₋₈ cycloalkyls or substituted cycloalkyls, etc.

For purpose of the present invention, the term "substituted" shall be understood to include adding or replacing one or more atoms contained within a functional group or compound with one or more different atoms.

For purposes of the present invention, substituted alkyls include carboxyalkyls, aminoalkyls, dialkylaminos, hydroxyalkyls and mercaptoalkyls; substituted cycloalkyls include moieties such as 4-chlorocyclohexyl; aryls include moieties such as napthyl; substituted aryls include moieties such as 3-bromophenyl; aralkyls include moieties such as toluyl; heteroalkyls include moieties such as ethylthiophene; substituted heteroalkyls include moieties such as 3-methoxy-thiophene; alkoxy includes moieties such as methoxy; and phenoxy includes moieties such as 3-nitrophenoxy. Halo- shall be understood to include fluoro, chloro, iodo and bromo.

The term "sufficient amounts" for purposes of the present invention shall mean an amount which achieves a therapeutic effect as such effect is understood by those of ordinary skill in the art.

One of the chief advantages of the compounds of the present invention is that the prodrugs have a higher payload per unit of polymer than previous techniques. It is generally preferred that the polymeric first releases the trimethyl lock (TML) based prodrug intermediate by hydrolysis and then the resultant intermediate or "second prodrug" moiety undergoes lactonization to regenerate, for example, a moiety which is either a biologically active compound or a composition comprising a further prodrug. The high payload polymeric conjugates of the present invention are thus unique delivery systems which can contain up to four or a greater number of molecules of a drug.

Methods of making and using the compounds and conjugates described herein are also provided.

### BRIEF DESCRIPTION OF THE DRAWINGS

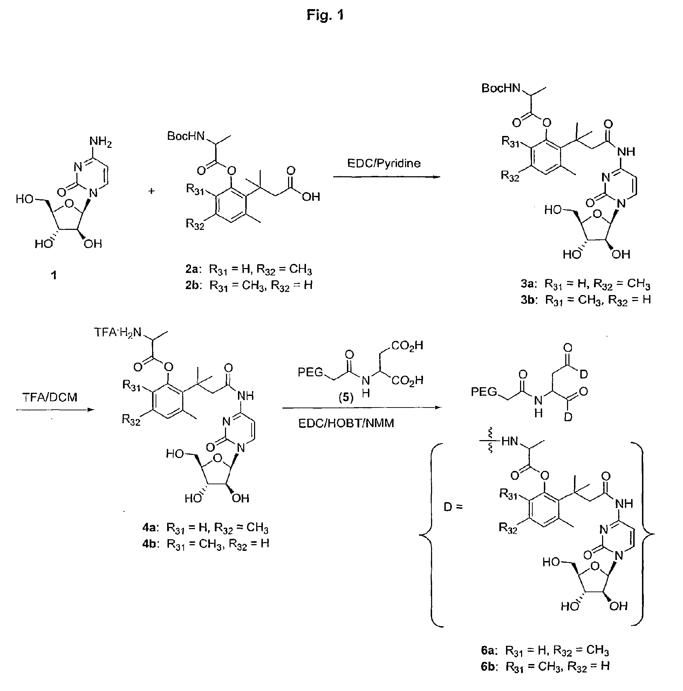

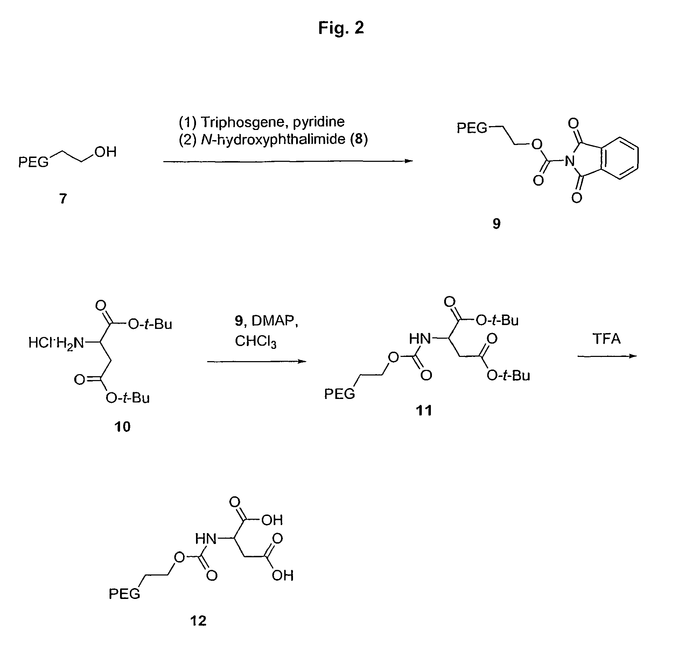

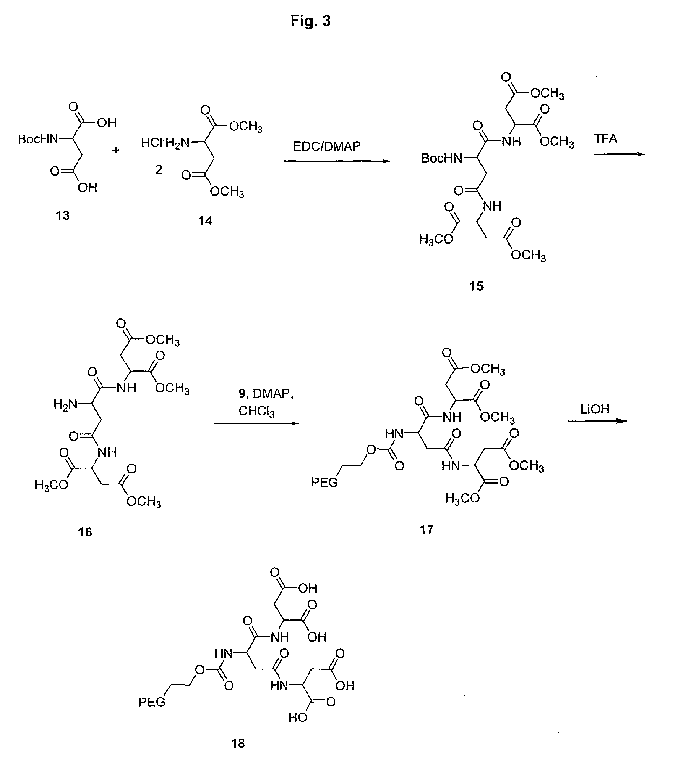

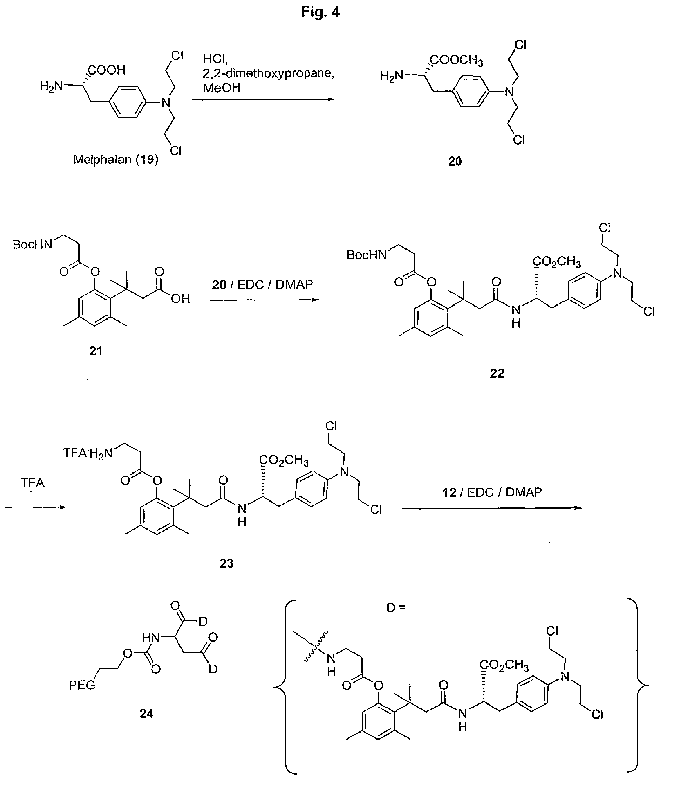

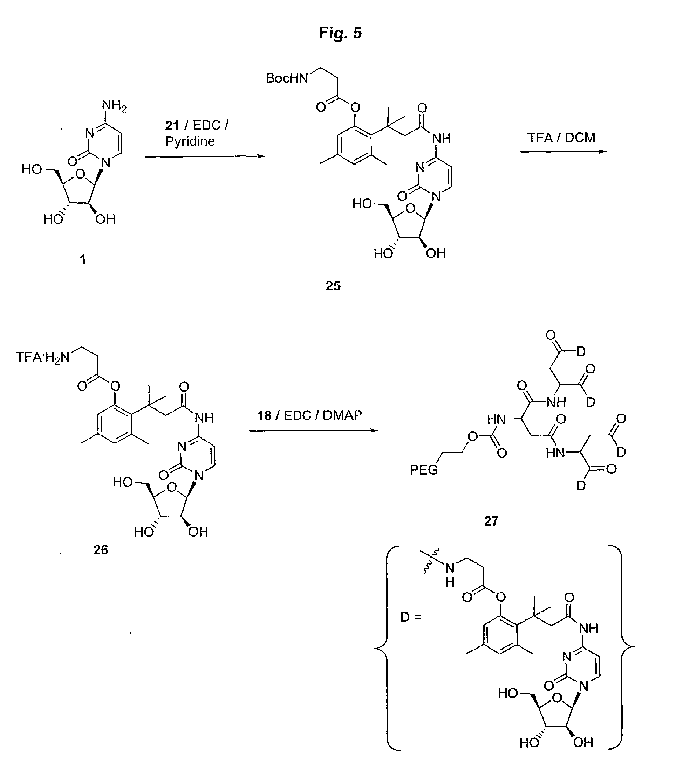

Figures 1- 5 schematically illustrate methods of forming compounds of the present invention which are described in the Examples.

### DETAILED DESCRIPTION OF THE INVENTION

### A. FORMULA (I)

In one preferred embodiment of the invention, there are provided compounds of the formula: 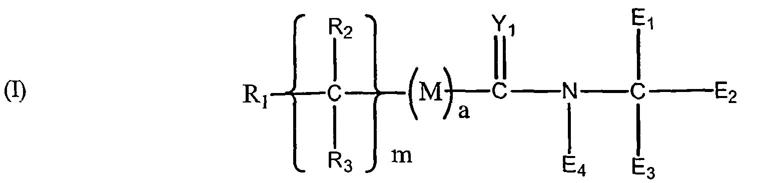 wherein:
R₁ is a polymeric residue;
Y₁ is O, S or NR₄;
M is O, S or NR₅;
(a) is zero or one;
(m) is zero or a positive integer;
E₁ is 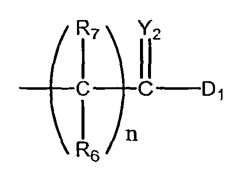
E₂₋₄ are independently H, E₁ or 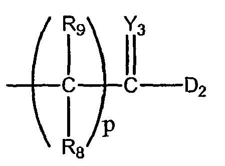
(n) and (p) are independently 0 or a positive integer;
Y₂₋₃ are independently O, S or NR₁₀;
R₂₋₁₀ are independently selected from the group consisting of hydrogen, C₁₋₆ alkyls, C₃₋₁₂ branched alkyls, C₃₋₈ cycloalkyls, C₁₋₆ substituted alkyl, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
D₁ and D₂ are independently OH, 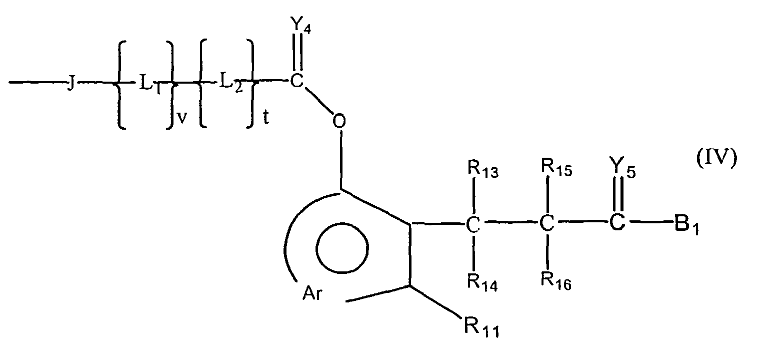 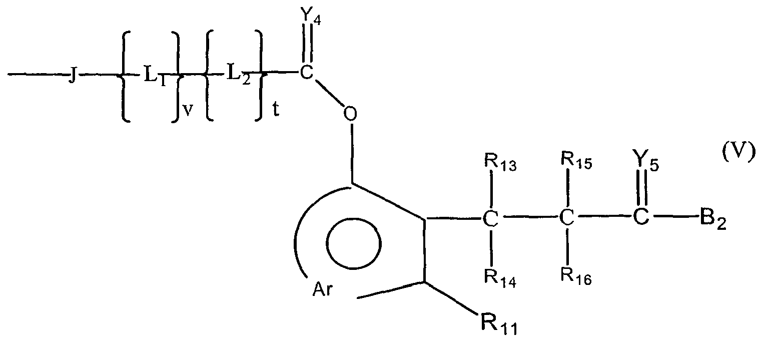
wherein
J is NR₁₂ or 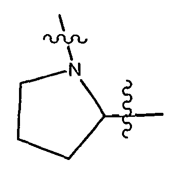 v) and (t) are independently 0 or a positive integer up to about 6 and preferably about1;
L₁ and L₂ are independently selected bifunctional linkers;
Y₄₋₅ are independently selected from the group consisting of O, S and NR₁₇;
R₁₁₋₁₇ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
Ar is a moiety which when included in Formula (I) forms a multi-substituted aromatic hydrocarbon or a multi-substituted heterocyclic group; and
B₁ and B₂ are preferably independently selected from among leaving groups, OH, residues of hydroxyl-containing moieties or residues of amine-containing moieties.

In another preferred embodiment, D₁ and D₂ are independently selected terminal branching groups of formula (VI) 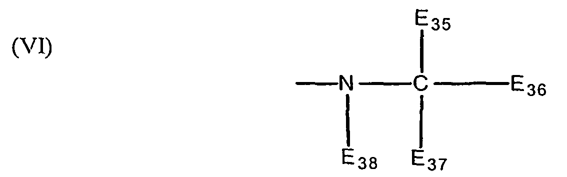 wherein:
E₃₅₋₃₈ are selected from the same group which defines E₁₋₄ above, except that within the definition, D₁ and D₂ are changed to D'₁ and D'₂ which are defined below. Within this embodiment, D'₁ and D'₂ can be independently OH, a moiety of formula (IV) or (V), or 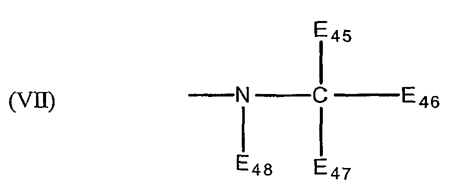
wherein E₄₅₋₄₈ are selected from the same group which defines E₁₋₄, except that within the definition D₁ and D₂ are changed to D"₁ and D"₂ and D"₁ and D"₂ independently OH, formula (IV) or formula (V). As can be appreciated from the above, when the terminal branching is taken to its fullest extent with a bifunctional polymer R₁, up to sixteen (16) equivalents of drug can be loaded onto the polymeric platform.

In those aspects of this embodiment where bis-substituted polymeric residues are desired, some preferred polymeric transport systems of the invention are shown below as formula (III): 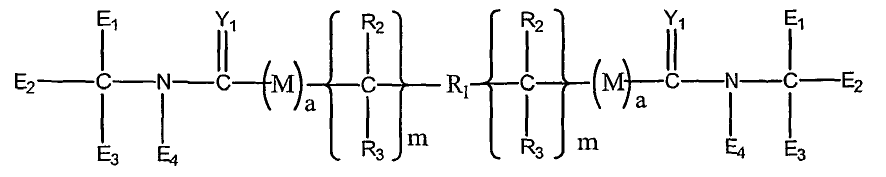 wherein all variables are as previously described.

The multi-loading polymer transport system of the present invention is based in large part on the polymeric residue designated herein as R₁. Optionally, R₁ includes a capping group A. The polymer capping group A includes, for example, moieties such as hydrogen, CO₂H, C₁₋₆ alkyl moieties, and compounds of formula (II) shown below, which forms a bis-system: 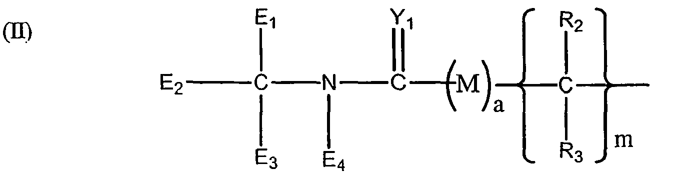 wherein all variables are as previously described. It will be understood and appreciated that the multiple terminal branching described above applies equally in the bis-systems as well.

With regard to the other variables which comprise the formulae of the present invention, the following are preferred:
Y₁₋₅ are each oxygen;
R₂₋₁₀ and R₁₂ are each preferably hydrogen or lower alkyl, e.g. C₁₋₆;
R₁₁, R₁₃ and R₁₄ are preferably -CH₃;
(m) is 1 or 2;
(n) and (p) are each either zero or an integer from 1-4;
(v) is zero or 1;
(t) is 1;
L₁ is -(CH₂CH₂O)₂-; and
L₂ is one of -CH₂-, -CH(CH₃)-, -(CH₂)₂-, -(CH₂)₂-NH-, -CH₂C(O)NHCH(CH₃)-, -(CH₂)₂-NH-, -CH₂C(O)NHCH₂-, -(CH₂)₂-NH-C(O)(CH₂)₂NH- or -CH₂C(O)NHCH(CH₂CH(CH₃)₂)-.

### B. DESCRIPTION OF THE Ar MOIETY

Referring to Formula (I), it can be seen that the Ar is a moiety, which when included in Formula (I), forms a multi-substituted aromatic hydrocarbon or a multi-substituted heterocyclic group. A key feature is that the Ar moiety is aromatic in nature. Generally, to be aromatic, the π electrons must be shared within a "cloud" both above and below the plane of a cyclic molecule. Furthermore, the number of π electrons must satisfy the Hückel rule (4n+2). Those of ordinary skill will realize that a myriad of moieties will satisfy the aromatic requirement of the moiety and thus are suitable for use herein. One particularly preferred aromatic group is: 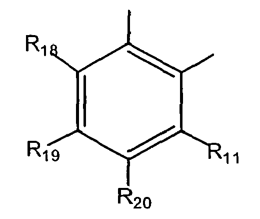 wherein R₁₈₋₂₀ are selected from the same group which defines R₁₁. Alternative aromatic groups include: 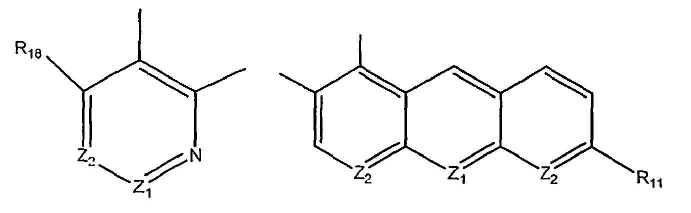 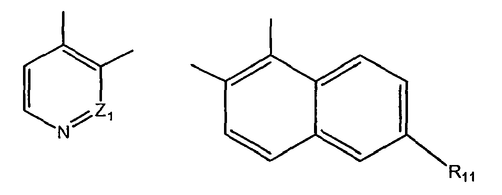 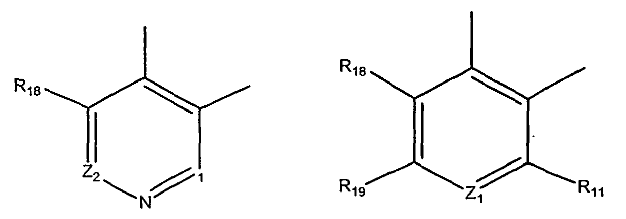 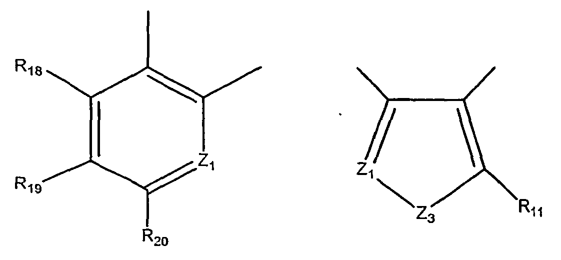 wherein and Z₁ and Z₂ are independently CR₂₂ or NR₂₁; and Z₃ is O, S or NR₂₁ where R₁₈₋₂₂ are selected from the same group as that which defines R₁₁ or a cyano, nitro, carboxyl, acyl, substituted acyl or carboxyalkyl. Isomers of the five and six-membered rings are also contemplated as well as benzo- and dibenzo- systems and their related congeners are also contemplated. It will also be appreciated by the artisan of ordinary skill that the aromatic rings can optionally be substituted with hetero-atoms such as O, S, NR₂₁, etc. so long as Hückel's rule is obeyed. Furthermore, the aromatic or heterocyclic structures may optionally be substituted with halogen(s) and/or side chains as those terms are commonly understood in the art. However, all structures suitable for Ar moieties of the present invention are capable of allowing the B₁, or B₂-containing moieties and the (R₁₁) moiety to be in an *ortho* arrangement with the same plane.

### C. DRUG GENERATION VIA HYDROLYSIS OF THE PRODRUG

The prodrug compounds of the present invention are designed so that the t_{1/2} of hydrolysis is < t_{1/2} elimination in plasma.

The linkages included in the compounds have hydrolysis rates in the plasma of the mammal being treated which is short enough to allow sufficient amounts of the parent compounds, i.e. the amino- or hydroxyl-containing bioactive compound, to be released prior to elimination. Some preferred compounds of the present invention have a t_{1/2} for hydrolysis in plasma ranging from about 5 minutes to about 12 hours. Preferably, the compositions have a plasma t_{1/2} hydrolysis ranging from about 0.5 to about 8 hours and most preferably from about 1 to about 6 hours.

### D. SUBSTANTIALLY NON-ANTIGENIC POLYMERS

As stated above, R₁ is a water soluble polymeric residue which is preferably substantially non-antigenic such as a polyalkylene oxide (PAO) or polyethylene glycol (PEG). In preferred aspects of the invention, R₁ further includes the previously mentioned capping group, designated herein as A, which allows a bifunctional or bis-polymer system to be formed.

As an example, the PEG residue portion of the inventive compositions can be selected from the following non-limiting list:

-C(=Y₆)-(CH₂)_{f}-O-(CH₂CH₂O)ₓ-A,

-C(=Y₆)-Y₇-(CH₂)_{f}-O-(CH₂CH₂O)ₓ-A,

-C(=Y₆)-NR₂₃-(CH₂)_{f}-O-(CH₂CH₂O)ₓ-A,

-(CR₂₄R₂₅)ₑ-O-(CH₂)_{f}-O-(CH₂CH₂O)ₓ-A,

-NR₂₃-(CH₂)_{f}-O-(CH₂CH₂O)ₓ-A,

-C(=Y₆)-(CH₂)_{f}-O-(CH₂CH₂O)ₓ-(CH₂)_{f}-C(=Y₆)-,

-C(=Y₆)-Y₇-(CH₂)_{f}-O-(CH₂CH₂O)ₓ-(CH₂)_{f}-Y₇-C(=Y₆)-,

-C(=Y₆)-NR₂₃-(CH₂)_{f}-O-(CH₂CH₂O)ₓ-(CH₂)_{f}-NR₂₃-C(=Y₆)-,

-(CR₂₄R₂₅)ₑ-O-(CH₂)_{f}-O-(CH₂CH₂O)ₓ-(CH₂)_{f}-O-(CR₂₄R₂₅)ₑ-, and

-NR₂₃-(CH₂)_{f}-O-(CH₂CH₂O)ₓ-(CH₂)_{f}-NR₂₃-

wherein Y₆ and Y₇ are independently O, S or NR₂₃;
x is the degree of polymerization;
R₂₃, R₂₄ and R₂₅ are independently selected from among H, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
*e* and *f* are independently zero, one or two; and
A is a capping group.

The degree of polymerization for the polymer (x) can be from about 10 to about 2,300. This represents the number of repeating units in the polymer chain and is dependent on the molecular weight of the polymer. The (A) moiety is a capping group as defined herein, i.e. a group which is found on the terminal of the polymer and, in some aspects, can be selected from any of H, NH₂, OH, CO₂H, C₁₋₆ alkyls or other PEG terminal activating groups, as such groups are understood by those of ordinary skill.

Also useful are polypropylene glycols, branched PEG derivatives such as those described in commonly-assigned U.S. Patent No. 5,643,575, "star-PEG's" and multi-armed PEG's such as those described in Shearwater Polymers, Inc. catalog "Polyethylene Glycol Derivatives 1997-1998". The disclosure of each of the foregoing is incorporated herein by reference. It will be understood that the water-soluble polymer can be functionalized for attachment to the bifunctional linkage groups if required without undue experimentation.

In a further embodiment R₁ is optionally selected from among one or more of dextran, polyvinyl alcohols, carbohydrate-based polymers, hydroxypropylmethacrylamide, polyalkylene oxides, and/or copolymers thereof. See also commonly-assigned U.S. Patent No, 6,153,655, the contents of which are incorporated herein by reference.

In many aspects of the present invention, bis-activated polyethylene glycols are preferred when di-or more substituted polymer conjugates are desired. Alternatively, polyethylene glycols (PEG's), mono-activated, C₁₋₄ alkyl-terminated polyalkylene oxides (PAO's) such as mono-methyl-terminated polyethylene glycols (mPEG's) are preferred when mono-substituted polymers are desired.

In order to provide the desired hydrolyzable linkage, mono- or di-acid activated polymers such as PEG acids or PEG diacids can be used as well as mono- or di-PEG amines and mono- or di-PEG diols. Suitable PAO acids can be synthesized by first converting mPEG-OH to an ethyl ester followed by saponification. See also Gehrhardt, H., et al. Polymer Bulletin 18: 487 (1987) and Veronese, F.M., et al., J. Controlled Release 10; 145 (1989). Alternatively, the PAO-acid can be synthesized by converting mPEG-OH into a *t*-butyl ester followed by acid cleavage. See, for example, commonly assigned U.S. Patent No. 5,605,976. The disclosures of each of the foregoing are incorporated by reference herein.

Although PAO's and PEG's can vary substantially in average molecular weight, the polymer portion of the prodrug is at least about 20,000 weight average in most aspects of the invention. Preferably, R₁ has a weight average molecular weight of from about 20,000 to about 100,000 and more preferably from about 25,000 to about 60,000. The average molecular weight of the polymer selected for inclusion in the prodrug must be sufficient so as to provide sufficient circulation of the prodrug before hydrolysis of the linker.

The polymeric substances included herein are preferably water-soluble at room temperature. A non-limiting list of such polymers include polyalkylene oxide homopolymers such as polyethylene glycol (PEG) or polypropylene glycols, polyoxyethylenated polyols, copolymers thereof and block copolymers thereof, provided that the water solubility of the block copolymers is maintained.

As an alternative to PAO-based polymers, effectively non-antigenic materials such as dextran, polyvinyl alcohols, carbohydrate-based polymers, hydroxypropylmethacrylamide (HPMA), and copolymers thereof etc. and the like can be used if the same type of activation is employed as described herein for PAO's such as PEG. Those of ordinary skill in the art will realize that the foregoing list is merely illustrative and that all polymeric materials having the qualities described herein are contemplated. For purposes of the present invention, "effectively non-antigenic" and "substantially non-antigenic" shall be understood to include all polymeric materials understood in the art as being substantially non-toxic and not eliciting an appreciable immune response in mammals.

It will be clear from the foregoing that other polyalkylene oxide derivatives of the foregoing, such as the polypropylene glycol acids, etc., as well as other bi- functional linking groups are also contemplated.

### E. PRODRUG CANDIDATES

### 1. Residues of Hydroxyl-containing Compounds

### a. Camptothecin and Related Topoisomerase I Inhibitors

Camptothecin is a water-insoluble cytotoxic alkaloid produced by *Camptotheca accuminata* trees indigenous to China and *nothapodytes foetida* trees indigenous to India. Camptothecin and related compounds and analogs are also known to be potential anticancer or antitumor agents and have been shown to exhibit these activities in vitro and in vivo. Camptothecin and related compounds are also candidates for conversion to the prodrugs of the present invention.

### Camptothecin and certain related analogues share the structure:

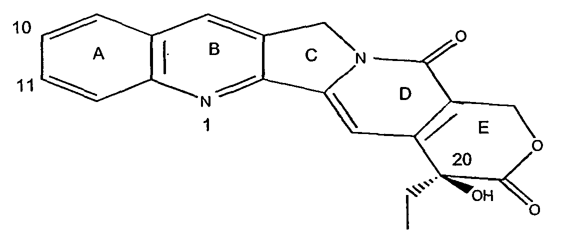

From this core structure, several known analogs have been prepared. For example, the A ring in either or both of the 10- and 11-positions can be substituted with an OH. The A ring can also be substituted in the 9-position with a straight or branched C₁₋₃₀ alkyl or C₁₋₁₇ alkoxy, optionally linked to the ring by a heteroatom i.e.- O or S. The B ring can be substituted in the 7-position with a straight or branched C₁₋₃₀ alkyl or substituted alkyl-, C₅₋₈ cycloakyl, C₁₋₃₀ alkoxy, phenyl alkyl, etc., alkyl carbamate, alkyl carbazides, phenyl hydrazine derivatives, amino-, aminoalkyl-, aralkyl, etc. Other substitutions are possible in the C, D and E rings. See, for example, U.S. Patent Nos. 5,004,758; 4,943,579; Re 32,518, the contents of which are incorporated herein by reference. Such derivatives can be made using known synthetic techniques without undue experimentation. Preferred camptothecin derivatives for use herein include those which include a 20-OH or another OH moiety which is capable of reacting directly with activated forms of the polymer transport systems described herein or to the linking moiety intermediates, e.g. iminodiacetic acid, etc., which are then attached to a polymer such as PEG.

### Reference to camptothecin analogs herein has been made for purposes of illustration and not limitation.

### b. Taxanes and Paclitaxel Derivatives

One class of compounds included in the prodrug compositions of the present invention is taxanes. For purposes of the present invention, the term "taxane" includes all compounds within the taxane family of terpenes. Thus, taxol (paclitaxel), 3'-substituted tert-butoxy-carbonyl-amine derivatives (taxoteres) and the like as well as other analogs which are readily synthesized using standard organic techniques or are available from commercial sources such as Sigma Chemical of St. Louis, Missouri are within the scope of the present invention. These derivatives have been found to be effective anti-cancer agents. Numerous studies indicate that the agents have activity against several malignancies. To date, their use has been severely limited by, among other things, their short supply, poor water solubility and a tendency to cause hypersensitivity. It is to be understood that other taxanes including the 7-aryl-carbamates and 7-carbazates disclosed in commonly assigned U.S. Patent Nos. 5,622,986 and 5,547,981 can also be included in the prodrugs of the present invention. The contents of the foregoing U.S. patents are incorporated herein by reference. Paclitaxel is a preferred taxane.

### c. Additional Biologically-Active Moieties

In addition to the foregoing molecules, the prodrug formulations of the present invention can be prepared using many other compounds. For example, biologically-active compounds such as bis-PEG conjugates derived from compounds such as gemcitabine: 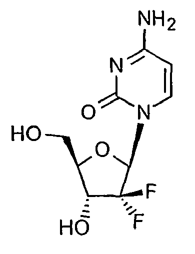 or podophyllotoxin: 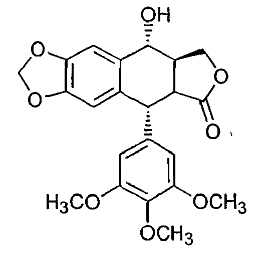 or triazole-based antifungal agents such as fluconazole: 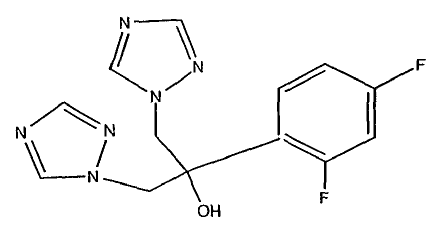 or ciclopirox: 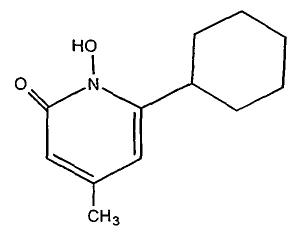 or Ara-C:

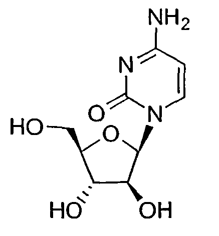

The parent compounds selected for prodrug forms need not be substantially water-insoluble, although the polymer-based prodrugs of the present invention are especially well suited for delivering such water-insoluble compounds. Other useful parent compounds include, for example, certain low molecular weight biologically active proteins, enzymes and peptides, including peptido glycans, as well as other anti-tumor agents; cardiovascular agents such as forskolin; anti-neoplastics such as combretastatin, vinblastine, doxorubicin, maytansine, etc.; anti-infectives such as vancomycin, erythromycin, etc.; anti-fungals such as nystatin, amphotericin B, triazoles, papulocandins, pneumocandins, echinocandins, polyoxins, nikkomycins, pradimicins, benanomicins, etc. see, "Antibiotics That Inhibit Fungal Cell Wall Development" Annu. Rev. Microbiol. 1994, 48:471-97, the contents of which are incorporated herein by reference; anti-anxiety agents, gastrointestinal agents, central nervous system-activating agents, analgesics, fertility or contraceptive agents, anti-inflammatory agents, steroidal agents, anti-urecemic agents, cardiovascular agents, vasodilating agents, vasoconstricting agents and the like.

The foregoing is illustrative of the biologically active moieties which are suitable for the prodrugs of the present invention. It is to be understood that those biologically active materials not specifically mentioned but having suitable ester-forming groups, i.e. hydroxyl moieties, are also intended and are within the scope of the present invention. It is also to be understood that the prodrug conjugates of the present invention may also include minor amounts of compounds containing not only one equivalent of drug and polymer but also a moiety which does not effect bioactivity in vivo. For example, it has been found that in some instances, in spite of reacting diacids with drug molecules having a single linkage point, the reaction conditions do not provide quantitative amounts of prodrugs with two equivalents of drug per polymer. By-products of the reactants can sometimes be formed such as acyl ureas if carbodiimides are used.

### 2. Residues of Amine-containing Compounds

In some aspects of the invention, B₁ or B₂ is a residue of an amine-containing compound, anon-limiting list of such suitable compounds include residues of organic compounds, enzymes, proteins, polypeptides, etc. Organic compounds include, without limitation, moieties such as anthracycline compounds including daunorubicin, doxorubicin; *p*-aminoaniline mustard, melphalan, Ara-C (cytosine arabinoside) and related anti-metabolite compounds, e.g., gemcitabine, etc. Alternatively, B can be a residue of an amine-containing cardiovascular agent, anti-neoplastic, anti-infective, antifungal such as nystatin and amphotericin B, anti-anxiety agent, gastrointestinal agent, central nervous system-activating agent, analgesic, fertility agent, contraceptive agent, anti-inflammatory agent, steroidal agent, anti-urecemic agent, vasodilating agent, vasoconstricting agent, etc.

In a preferred aspect of the invention, the amino-containing compound is a biologically active compound that is suitable for medicinal or diagnostic use in the treatment of animals, e.g., mammals, including humans, for conditions for which such treatment is desired. The foregoing list is meant to be illustrative and not limiting for the compounds which can be modified. Those of ordinary skill will realize that other such compounds can be similarly modified without undue experimentation. It is to be understood that those biologically active materials not specifically mentioned but having suitable amino-groups are also intended and are within the scope of the present invention.

The only limitations on the types of amino-containing molecules suitable for inclusion herein is that there is available at least one (primary or secondary) amine-containing position which can react and link with a carrier portion and that there is not substantial loss of bioactivity after the prodrug system releases and regenerates the parent compound.

It is noted that parent compounds suitable for incorporation into the prodrug compositions of the invention, may themselves be substances/compounds which are not active after hydrolytic release from the linked composition, but which will become active after undergoing a further chemical process/reaction. For example, an anticancer drug that is delivered to the bloodstream by the double prodrug transport system, may remain inactive until entering a cancer or tumor cell, whereupon it is activated by the cancer or tumor cell chemistry, e.g., by an enzymatic reaction unique to that cell.

### 3. Leaving Groups

In those aspects where B₁ or B₂ is a leaving group, suitable leaving groups include, without limitations, moieties such as N-hydroxybenzotriazolyl, halogen, N-hydroxyphthalimidyl, p-nitrophenoxy, imidazolyl, N-hydroxysuccinimidyl; thiazolidinyl thione, or other good leaving groups as will be apparent to those of ordinary skill. The synthesis reactions used and described herein will be understood by those of ordinary skill without undue experimentation.

For example, an acylated intermediate of compound (I) can be reacted with a reactant such as 4-nitrophenyl chloroformate, disuccinimidyl carbonate (DSC), carbonyldiimidazole, thiazolidine thione, etc. to provide the desired activated derivative.

The selective acylation of the phenolic or anilinic portion of the p-hydroxybenzyl alcohol or the p-aminobenzyl alcohol and the o-hydroxbenzyl alcohol or the o-aminobenzyl alcohol can be carried out with, for example, thiazolidine thione activated polymers, succinimidyl carbonate activated polymers, carboxylic acid activated polymers, blocked amino acid derivatives. Once in place, the "activated" form of the PEG prodrug (or blocked prodrug) is ready for conjugation with an amine- or hydroxyl-containing compound.

### F. SYNTHESIS OF THE POLYMERIC PRODRUG TRANSPORT SYSTEM

Synthesis of representative polymer prodrugs is set forth in the Examples. Generally, however, in one preferred method of preparing the prodrug transport systems, the polymer residue is first attached to the branching groups. Separately, the biologically active moiety or drug, e.g. Drug-OH or Drug-NH₂ (B₁ or B₂ of formula I) is attached to the TML component which may also include a bifunctional spacer thereon at point of attachment to the polymer. Next, the polymeric residue containing the terminal branches is reacted with the drug-TML portion under conditions sufficient to form the final product.

Attachment of the bifunctional spacer containing the TML-Drug component to the polymer portion is preferably carried out in the presence of a coupling agent. A non-limiting list of suitable coupling agents include 1,3-diisopropylcarbodiimide (DIPC), any suitable dialkyl carbodiimides, 2-halo-1-alkyl-pyridinium halides, (Mukaiyama reagents), 1-(3-dimethylaminopropyl)-3-ethyl carbodiimide (EDC), propane phosphonic acid cyclic anhydride (PPACA) and phenyl dichlorophos-phates, etc. which are available, for example from commercial sources such as Sigma-Aldrich Chemical, or synthesized using known techniques.

Preferably the substituents are reacted in an inert solvent such as methylene chloride, chloroform, DMF or mixtures thereof. The reaction also preferably is conducted in the presence of a base, such as dimethylaminopyridine, diisopropylethylamine, pyridine, triethylamine, etc. to neutralize any acids generated and at a temperature from 0°C up to about 22°C (room temperature).

More particularly, one method of preparing a polymeric transport system includes reacting a compound of the formula (VIII): 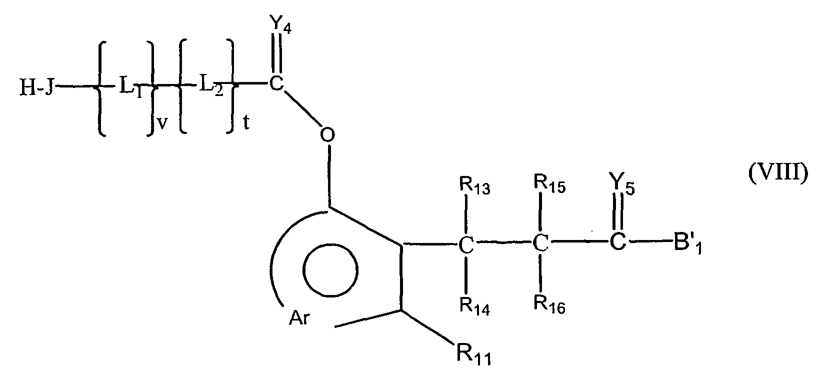 wherein all variables are as previously defined and
B'₁ is a residue of a hydroxyl- or an amine-containing moiety;
with a compound of the formula (IX): 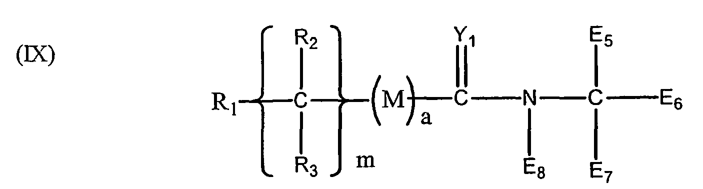 wherein
R₁ is a polymeric residue; Y₁ is O, S or NR₄; M is O, S or NR₅; (a) is zero or one; (m) is 0 or a positive integer; Y₂₋₃ are independently O, S or NR₁₀; and R₂₋₃ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
E₅ is 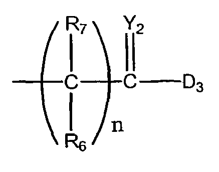 E₆₋₈ are independently H, E₅ or 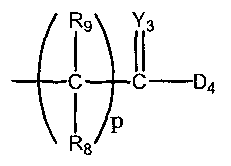 wherein D₃ and D₄ are independently OH or a leaving group which is capable of reacting with an unprotected amine or hydroxyl or a terminal branching group;
(n) and (p) are independently 0 or a positive integer;
Y₂₋₃ are independently O, S or NR₁₀; and
R₆₋₁₀ are independently selected from the group consisting of hydrogen, C₁₋₆ alkyls, C₃₋₁₂ branched alkyls, C₃₋₈ cycloalkyls, C₁₋₆ substituted alkyl, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;

In further aspects of the method, D₃ and D₄ are independently selected terminal branching groups of formula (X) 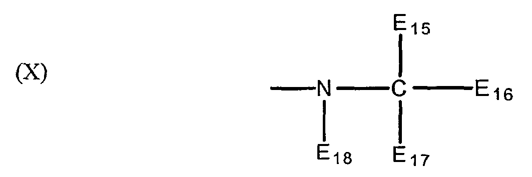 where E₁₅₋₁₈ are selected from the same group which defines E₅₋₈, except that D₃ and D₄ are changed to D'₃ and D'₄ which are defined below. Within this embodiment, D'₃ and D'₄ can be independently OH, a moiety of formula (IV) or (V), or (XI) 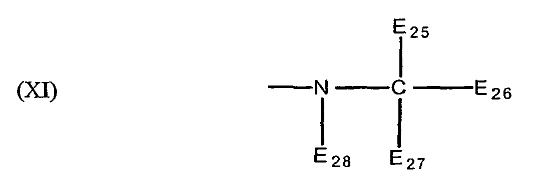 wherein E₂₅₋₂₈ are selected from the same group which defines E₅-₈, except that D₃ and D₄ are changed to D"₃ and D"₄ which are defined as being independently OH or a leaving group which is capable of reacting with an unprotected amine or hydroxyl.

Such synthetic techniques allow up to sixteen (16) equivalents of carboxylic acid or activated carboxylic acid, for example, to be attached. As shown in the preferred structures herein, PEG residues with terminally branched multi-acids are preferred aspects of the invention.

Regardless of the synthesis selected, some of the preferred compounds which result from the synthesis techniques described herein include: 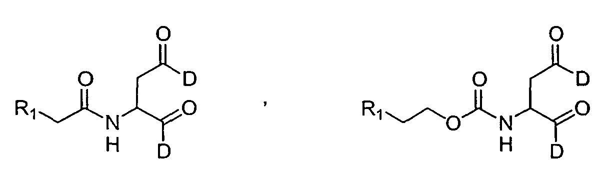 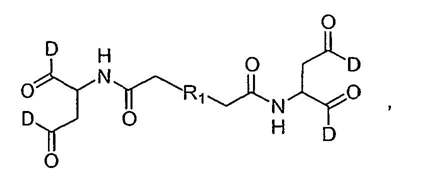 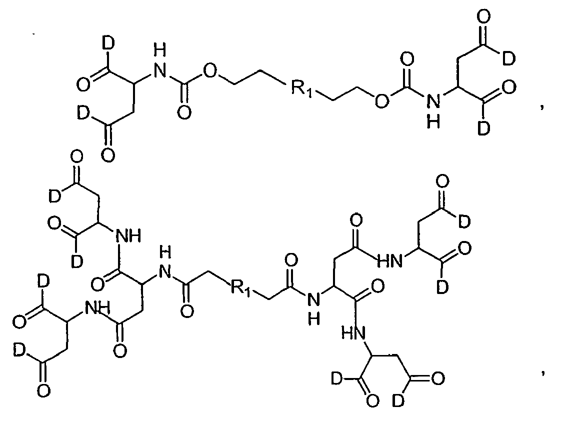 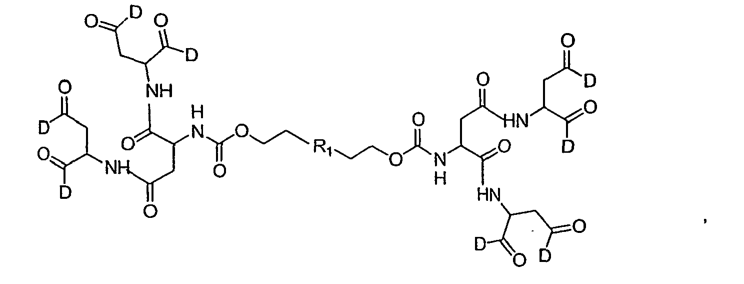 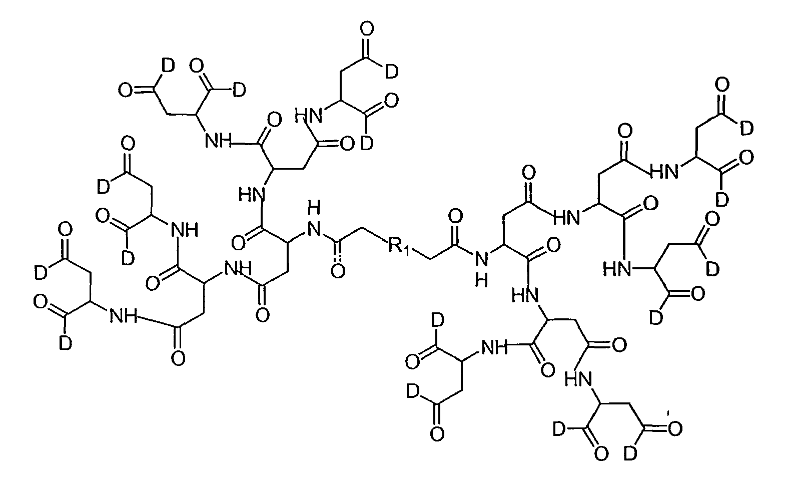 and 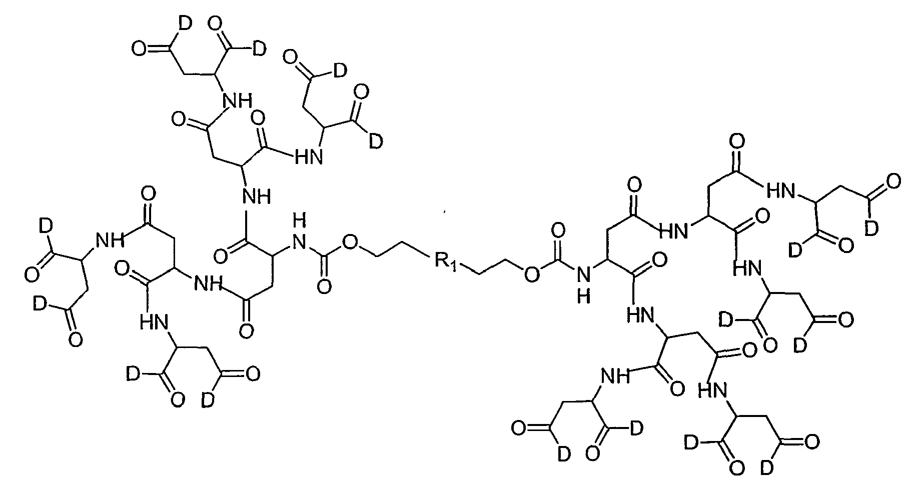 wherein R₁ is a polymer residue such as a PAO or PEG and D is OH, formula (IV) or (V). Preferably, D is 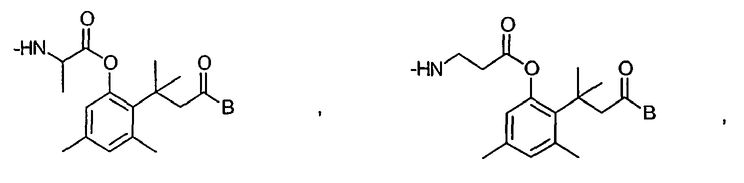 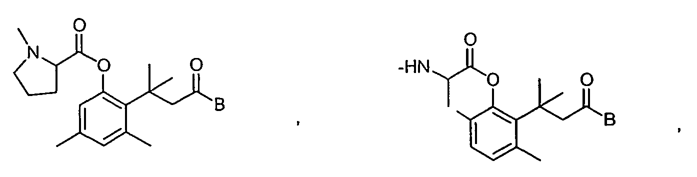 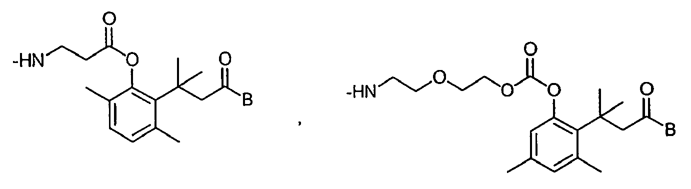 or 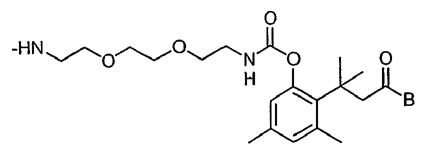 where B is a residue of an amine or a hydroxyl- containing drug.

In another preferred aspect of the invention, the compounds of the present invention are of formula (XII): 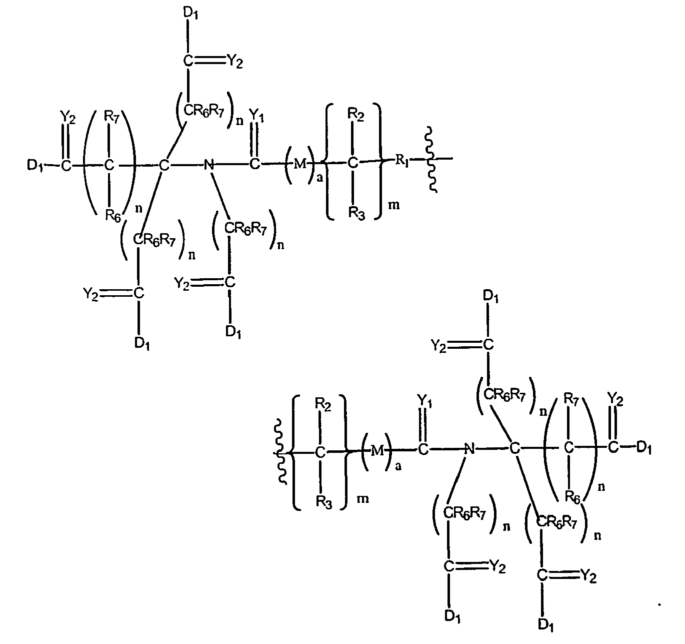 wherein all variables are as previously defined above.

### G. IN VIVO DIAGNOSTICS

A further aspect of the invention provides the conjugates of the invention optionally prepared with a diagnostic tag linked to the transport enhancer described above, wherein the tag is selected for diagnostic or imaging purposes. Thus, a suitable tag is prepared by linking any suitable moiety, e.g., an amino acid residue, to any art-standard emitting isotope, radio-opaque label, magnetic resonance label, or other non-radioactive isotopic labels suitable for magnetic resonance imaging, fluorescence-type labels, labels exhibiting visible colors and/or capable of fluorescing under ultraviolet, infrared or electrochemical stimulation, to allow for imaging tumor tissue during surgical procedures, and so forth. Optionally, the diagnostic tag is incorporated into and/or linked to a conjugated therapeutic moiety, allowing for monitoring of the distribution of a therapeutic biologically active material within an animal or human patient.

In a still further aspect of the invention, the inventive tagged conjugates are readily prepared, by art-known methods, with any suitable label, including, e.g., radioisotope labels. Simply by way of example, these include ¹³¹Iodine, ¹²⁵Iodine, ^{99m}Technetium and/or ¹¹¹Indium to produce radioimmunoscintigraphic agents for selective uptake into tumor cells, *in vivo*. For instance, there are a number of art-known methods of linking peptide to Tc-99m, including, simply by way of example, those shown by U.S. Patent Nos. 5,328,679; 5,888,474; 5,997,844; and 5,997,845, incorporated by reference herein.

Broadly, for anatomical localization of tumor tissue in a patient, the conjugate tag is administered to a patient or animal suspected of having a tumor. After sufficient time to allow the labeled immunoglobulin to localize at the tumor site(s), the signal generated by the label is detected, for instance, visually, by X-ray radiography, computerized transaxial tomography, MRI, by instrumental detection of a luminescent tag, by a photo scanning device such as a gamma camera, or any other method or instrument appropriate for the nature of the selected tag.

The detected signal is then converted to an image or anatomical and/or physiological determination of the tumor site. The image makes it possible to locate the tumor *in vivo* and to devise an appropriate therapeutic strategy. In those embodiments where the tagged moiety is itself a therapeutic agents, the detected signal provides evidence of anatomical localization during treatment, providing a baseline for follow-up diagnostic and therapeutic interventions.

### H. METHODS OF TREATMENT

Another aspect of the present invention provides methods of treatment for various medical conditions in mammals. The methods include administering to the mammal in need of such treatment, an effective amount of a prodrug, such as a multi-loaded Ara-C-PEG conjugates, which has been prepared as described herein. The compositions are useful for, among other things, treating neoplastic disease, reducing tumor burden, preventing metastasis of neoplasms and preventing recurrences of tumor/neoplastic growths in mammals.

The amount of the prodrug administered will depend upon the parent molecule included therein. Generally, the amount of prodrug used in the treatment methods is that amount which effectively achieves the desired therapeutic result in mammals. Naturally, the dosages of the various prodrug compounds will vary somewhat depending upon the parent compound, rate of in vivo hydrolysis, molecular weight of the polymer, etc. In general, however, prodrug taxanes are administered in amounts ranging from about 5 to about 500 mg/m² per day, based on the amount of the taxane moiety. Camptothecin prodrugs are also administered in amounts ranging from about 5 to about 500 mg/m² per day. The range set forth above is illustrative and those skilled in the art will determine the optimal dosing of the prodrug selected based on clinical experience and the treatment indication. Actual dosages will be apparent to the artisan without undue experimentation.

The prodrugs of the present invention can be included in one or more suitable pharmaceutical compositions for administration to mammals. The pharmaceutical compositions may be in the form of a solution, suspension, tablet, capsule or the like, prepared according to methods well known in the art. It is also contemplated that administration of such compositions may be by the oral and/or parenteral routes depending upon the needs of the artisan. A solution and/or suspension of the composition may be utilized, for example, as a carrier vehicle for injection or infiltration of the composition by any art known methods, e.g., by intravenous, intramuscular, subdermal injection and the like.

Such administration may also be by infusion into a body space or cavity, as well as by inhalation and/or intranasal routes. In preferred aspects of the invention, however, the prodrugs are parenterally administered to mammals in need thereof.

### I. EXAMPLES

The following examples serve to provide further appreciation of the invention but are not meant in any way to restrict the effective scope of the invention. The underlined and bold-faced numbers recited in the Examples correspond to those shown in **Figures 1-5.**
**General.** All reactions were run under an atmosphere of dry nitrogen or argon. Commercial reagents were used without further purification. All PEG compounds were dried under vacuum or by azeotropic distillation (toluene) prior to use. ¹H spectra were obtained with a JEOL FT NMR System JNM GSX-270 instrument using deuteriochloroform as solvent unless specified. ¹³C NMR spectra were obtained at 67.80 MHz on the JNM GSX-270. Chemical shifts (δ) are reported in parts per million (ppm) downfield from tetramethylsilane (TMS) and coupling constants (J values) are given in hertz (Hz). All PEG conjugated compounds were dissolved (∼15 mg/mL) in sterile saline (0.9%) for injection prior to *in vivo* drug treatments and were given as their ara-C equivalents (absolute amount of ara-C given).
**HPLC Method.** Analytical HPLC's were performed using a C8 reversed phase column (Beclanan, ultrasphere) under isocratic conditions with an 80:20 mixture (v/v) of methanol-water as mobile phase. Peak elutions were monitored at 254 nm using a UV detector. To detect the presence of any free PEG and also to confirm the presence of PEGYLATED product, an evaporative light scattering detector (ELSD), Model PL-EMD 950 (Polymer Laboratories), was employed. Based on ELSD and UV analysis, all the final PEGylated products were free of native drug and were ≥ 95% pure by HPLC. **Analysis of Ara-C Content in PEG Derivatives.** For the determination of the ara-C content in PEG derivatives, *N⁴*-acetylcytidine was used as a model. The UV absorbance of *N⁴*-acetylcytidine in H₂O was determined at 257 nm for six different concentrations ranging from 0.01 µmol/mL to 0.05 µmol/mL. From the standard plot of absorbance *vs*. concentration, the absorption coefficient, ε, of *N⁴*-acetylcytidine was calculated to be 36.4 (O.D. at 257 nm for 1 mg/mL with 1.0 cm light path). PEGylated ara-C derivatives were dissolved in H₂O at an approximate concentration of 0.015 µmol/mL (based on a MW of 40 kDa) and the UV absorbance of these compounds at 257 nm was determined. Using this value and employing the absorption coefficient, ε, obtained from the above, the concentration of ara-C in the sample was determined. Dividing this value by the sample concentration provided the percentage of ara-C in the sample.
**Analysis of Melphalan Content in PEG Derivatives.** For the determination of the melphalan content in PEG derivatives, melphalan was used as a standard. The UV absorbance of melphalan in DMF-H₂O (9:1, v/v) was determined at 264 nm for five different concentrations ranging from 0.02 µmol/mL to 0.06 µmol/mL. From the standard plot of absorbance *vs*. concentration, the absorption coefficient, ε, of melphalan was calculated to be 54.6 (O.D. at 264 nm for 1 mg/mL with 1.0 cm light path). PEGYLATED melphalan derivatives were dissolved in DMF-H₂O (9:1, v/v) at an approximate concentration of 0.013 µmol/mL (based on a MW of 40 kDa) and the UV absorbance of these compounds at 264 nm was determined. Using this value and employing the absorption coefficient, ε, obtained from the above, the concentration of melphalan in the sample was determined. Dividing this value by the sample concentration provided the percentage of melphalan in the sample.
**Abbreviations.** DCM (dichloromethane), DMAP (4-(dimethylamino)pyridine), EDC (1-ethyl-3-(3-dimethylaminopropyl)carbodiimide), HOBT (1-hydroxybenzotriazole), IPA (2-propanol), NMM (*N*-methylmorpholine), TFA (trifluoroacetic acid).

### Example 1.

**Compound 3a.** A mixture of ara-C (1, 1.73 g, 7.12 mmol), **2a** (700 mg, 1.78 mmol), HOBT (0.96 g, 7.12 mmol), and EDC●HCl (2.73 g, 14.25 mmol) in anhydrous pyridine (50 mL) was stirred at room temperature for 2 h , the temperature raised to 40 °C and the reaction continued overnight. The solvent was removed, methylene chloride (50 mL) was used to dissolve the mixture followed by washing with water (3 × 30 mL) and then with 0.1 N HCl (2 × 30 mL). The organic layer was dried over anhydrous MgSO_{4,} and the solvent removed *in vacuo* to give the crude product which was purified by silica gel column chromatography (5 to 10% MeOH in DCM) to give 638.8 mg (52%) of **3a** as a white solid: ¹H NMR δ 1.42, 1.55, 2.17, 2.26, 2.46, 2.79, 3.84, 3.91, 4.14, 4.33, 4.53, 5.49, 6.07, 6.17, 6.52, 6.76, 7.31, 7.67, 8.16, 8.62; ¹³C NMR δ 17.77, 20.11, 25.36, 28.32, 31.51, 31.96, 39.57, 50.18, 50.45, 61.88, 74.50, 80.15, 85.90, 88.58, 96.25, 122.51, 132.82, 133.34, 136.73, 138.22, 146.57, 149.90, 155.65, 155.96, 162.08, 171.89, 174.06.

### Example 2.

**Compound 3b.** Compound 1 was coupled with **2b** using a similar condition as in Example 1 to produce **3b** in 54% yield: ¹³C NMR δ_17.23, 17.92, 18.33, 25.49, 28.32, 31.51, 31.58, 31.99, 32.46, 39.52, 40.09, 50.08, 50.22, 61.72, 74.50, 74.94, 80.11, 80.15, 85.45, 85.90, 88.01, 88.58, 96.25, 122.51, 128.77, 129.03, 129.16, 131.68, 132.82, 136.24, 136.73, 138.22, 146.05, 146.57, 149.90, 155.65, 155.96, 171.85, 171.89, 174.06.

### Example 3.

**Compound 4a.** Compound **3a** (638.8 mg, 1.03 mmol) was stirred in anhydrous DCM (6 mL) and TFA (4 mL) at room temperature for 2 h. Ethyl ether was added to the solution to precipitate the crude product which was filtered and washed with ether to give **4a** as a white solid (534.5 mg, 82%): ¹H NMR (DMSO-*d₆*) δ 1.52 (s, 3H, (CH₃)₂CH) 1.55 (s, 3H, (CH₃)₂CH), 1.62 (d, 1 H, *J*= 8.1 Hz, (CH₃)₂CH), 2.22 (s, 3H, CH₃Ar), 2.57 (s, 3H, CH₃Ar), 2.97 (s, 2H, CH₂C(=O)), 3.41-4.27 (m, 5 H, ara-C's H-2'-H5'), 6.09 (d, 1H, *J* = 5.4, ara-C's H-1'), 6.67 (s, 1H, Ar-H), 6.90 (s, 1H, Ar-H), 7.12 (d, *J*= 5.4, H-6), 8.05 (d, *J* = 8.1, H-5), 8.67 (bs, 1H, TFA); ¹³C NMR (DMSO-*d₆*) δ 15.45, 19.67, 24.97, 31.05, 31.23, 38.56, 40.41, 48.53, 49.02, 61.02, 64.94, 74.64, 76.14, 85.74, 86.95, 94.32, 122.32, 132.41, 134.08, 135.67, 138.09, 146.71, 149.20, 154.50, 158.21, 158.72, 162.02, 169.68, 171.87.

### Example 4.

**Compound 4b.** Compound **3b** was subjected to the same condition as in Example 3 to give **4b** in 82% yield: ¹H NMR (DMSO-*d₆*) δ_1.52 (s, 3H, (CH₃)₂CH) 1.55 (s, 3H, (CH₃)₂CH), 1.62 (d, 1 H, *J*= 8.1 Hz, (CH₃)₂CH), 2.22 (s, 3H, CH₃Ar), 2.57 (s, 3H, CH₃Ar), 2.97 (s, 2H, CH₂C(=O)), 3.41-4.27 (m, 5 H, ara-C's H-2'-H5'), 6.09 (d, 1H, *J* = 5.4, ara-C's H-1'), 6.67 (s, 1H, Ar-H), 6.90 (s, 1H, Ar-H), 7.12 (d, *J*= 5.4, H-6), 8.05 (d, *J* = 8.1, H-5), 8.67 (bs, 1H, TFA); ¹³C NMR (DMSO-*d₆*) δ_15.45, 19.67, 24.97, 31.05, 31.23, 38.56, 40.41, 48.53, 49.02, 61.02, 64.94, 74.64, 76.14, 85.74, 86.95, 94.32, 122.32, 132.41, 134.08, 135.67, 138.09, 146.71, 149.20, 154.50, 158.21, 158.72, 162.02, 169.68, 171.87.

### Example 5.

**Compound 6a.** A mixture of PEG-aspartic acid (mw. 40,000, 5, 3 g, 0.074 mmol), **4a** (385.6 mg, 0.74 mmol), NMM (240 mg, 2.38 mmol), HOBT (120.5 mg, 0.89 mmol), and EDC●HCl (228.4 mg, 1.19 mmol) in anhydrous DCM (50 mL) was stirred at 0 °C for 30 minutes. The reaction was allowed to warm to room temperature and continued for 3 days and filtered. The filtrate was concentrated *in vacuo* and the residue recrystallized from IPA to give 2.7 g (90%) of product. The amount of ara-C in the product measured by UV assay was 2.11 wt%: ¹³C NMR δ 14.40, 19.22, 24.86, 31.17, 38.26, 8.90, 47.94, 48.67, 49.66, 60.17, 61.12, 61.90, 67.86-70.87 (PEG), 71.70, 74.50, 85.01, 87.53, 95.28, 121.39, 131.18, 132.68, 133.19, 134.77, 137.70, 145.26, 138.93, 155.23, 160.12, 161.56, 168.39, 170.72, 170.92, 171.27, 171.34.

### Example 6.

**Compound 6b.** Compound **4b** was subjected to the same condition as in Example 5 to give 6b in 88% yield. The amount of ara-C in the product measured by UV assay was 1.68 wt%: ¹³C NMR δ 15.12, 16.22, 24.52, 24.73, 29.55, 30.55, 31.15, 38.04, 38.59, 47.66, 49.16, 49.93, 50.18, 60.93, 61.12, 62.90, 69.44-71.59 (PEG), 71.70, 74.50, 84.78, 84.90, 87.53, 94.85, 127.60, 130.20, 135.51, 136.10, 141.70, 145.15, 147.50, 155.00, 161.20, 169.47, 170.62, 170.92, 171.27.

### Example 7.

**Compound 9.** PEG diol (7, 55 g, 1.38 mmol) was azeotroped in toluene over a 2 hour period followed by removal of 200 mL of solvent by rotary evaporation. The solution was cooled to ∼30 °C and triphosgene (0.544 g, 1.83 mmol) was added as solid followed by anhydrous pyridine (0.434 g, 5.49 mmol), and the reaction mixture stirred at 50 °C for 1 hour. *N*-hydroxyphthalimide (**8**, 1.12 g, 6.88 mmol) and anhydrous pyridine (0.54 g, 6.88 mmol) were added to the chloroformate mixture and the reaction stirred for a further 2 hours at 50 °C then for 12 hours at room temperature. The reaction mixture was filtered through filter paper and the solvent removed *in vacuo* and the product crystallized from methylene chloride-ethyl ether (1100 mL, 8:2, v/v) to give the product (50.9 g, 92%): ¹³C NMR δ 123.62, 128.10, 134.55, 152.00, 160.00.

### Example 8.

**PEG-cmc-Asp-O-*t*-Bu (11).** Compound **9** (mw. 40,000, 20 g, 0.459 mmol) and aspartic acid di *t*-butyl ester HCl (**10,** 1.0 g, 3.55 mmol) were dissolved in anhydrous DCM, followed by addition of DMAP (0.433 g, 3.55 mmol). The solution was refluxed overnight followed by precipitation by addition of ethyl ether (1 L). The solid was isolated by filtration and recrystallized from IPA (1 L) twice. The filter cake was washed with IPA (200 mL) and ether (200 mL) to give 15.6 g (78%) of product after drying at 45 °C *in vacuo*: ¹³C NMR δ 27.837 (CH₂CO₂C(*C*H₃)₃), 27.991 (CHCO₂C(*C*H₃)₃), 37.752 (CH*C*H₂CO₂), 50.800 (NHCH), 64.212 (OCH₂*C*H₂OC(=O)NH), 81.333 (CH₂CO₂*C*(CH₃)₃), 82.007 (CHCO₂*C*(CH₃)₃), 155.924 (OCH₂CH₂O*C*(=O)NH), 169.674 (CH₂*C*O₂C(CH₃)₃), 169.969 (CH*C*O₂C(CH₃)₃).

### Example 9.

**PEG-cmc-Asp-OH (12).** Compound **11** (15 g, 0.375 mmol) was dissolved in DCM (150 mL) followed by the addition of TFA (75 mL). The solution was stirred at room temperature for 2 hours and hexane (500 mL) added to precipitate the solid. The solid was triturated with hexane to remove TFA followed by recrystallization from chilled DCM-ether. The recrystallized solid was redissolved in DCM (150 mL) and washed with water (150 mL). The organic layer was separated, dried over anhydrous MgSO₄, concentrated *in vacuo*, and precipitated with ether to give 12.4 g (83%) of product: ¹³C NMR δ 36.441 (CH*C*H₂CO₂), 50.177 (NHCH), 64.390 (OCH₂*C*H₂OC(=O)NH), 81.333 (CH₂CO₂*C*(CH₃)₃), 82.007 (CHCO₂*C*(CH₃)₃), 156.172 (OCH₂CH₂O*C*(=O)NH), 171.944 (CH₂*C*O₂C(CH₃)₃), 172.211 (CH*CO*₂C(CH₃)₃).

### Example 10.

**Boc-Asp-Asp-OMe (15).** EDC●HCl (2.47 g, 12.86 mmol) was added to a mixture of BocNH-aspartic acid (**13**, 1 g, 4.29 mmol), aspartic acid dimethyl ester●HCl (14, 1.86 g, 9.43 mmol), and DMAP (2.47 g, 12.86 mmol) in anhydrous DCM (30 mL) and DMF (2 mL) at 0 °C. The mixture was allowed to warm up to room temperature overnight. The mixture was washed with 1N HCl three times and the organic layer was dried over anhydrous MgSO₄, followed by removal of the solvent *in vacuo* to give the product (2.0 g, 90%): ¹H NMR δ 1.45 (s, 9H), 2.62-3.02 (m, 6H, 3 × C*H*), 3.70 (s, 6H, 2 × OC*H*₃), 3.74 (s, 3H, OC*H*₃), 3.75 (s, 3H, OC*H*₃), 4.50 (bs, 1H, C*H*), 4.85 (m, 2H, 2 × C*H*), 6.05 (d, *J* = 6.95 Hz, 1H, N*H*), 6.98 (d, *J* = 8.05 Hz, 1H, N*H*), 7.57 (d, *J* = 7.69 Hz, 1H, N*H*).

### Example 11.

**Asp-Asp-OMe (16).** Compound **15** (2.0 g, 3.85 mmol) was dissolved in DCM (30 mL) and TFA (15 mL) and the solution was stirred for 2 h at room temperature. The solvent was removed *in vacuo* and the residue was recrystallized twice with DCM-ether to give the product (1.74 g, 87%) as a white solid: ¹³C NMR δ 35.52, 48.76, 50.12, 51.90, 51.96, 52.65, 114.59, 118.49, 168.43, 170..02, 170.92, 171.17, 171.40, 171.48.

### Example 12.

**PEG-cmc-Asp-Asp-OMe (17).** DMAP (4.5 g, 36.86 mmol) was added to a solution of **9** (mw. 40,000, 74 g, 1.84 mmol) and **16** (9.83 g, 18.43 mmol) in 700mL of anhydrous chloroform. The reaction mixture was refluxed for 24 hours under nitrogen. The reaction was cooled to room temperature and concentrated to ¼ volume. Crude product was precipitated with 2.5 L of ether, filtered and recrystallized from 5.5 L of IPA (65°C). The product was filtered and washed twice with fresh IPA, twice with fresh ether, and dried overnight at 40 °C to yield 59.0g (84%) of 17: ¹³C NMR δ 35.344, 36.931, 48.082, 48.208, 50.835, 51.509, 52.239, 61.045, 63.953, 68.854-72.056, 155.538, 170.102, 170.369, 170.453, 170.734.

### Example 13.

**PEG-cmc-Asp-Asp-OH (18).** Compound **17** (51 g, 1.26 mmol) and LiOH●H₂O (0.8 g, 18.9 mmol) were dissolved in 300 mL of water and the solution stirred overnight at room temperature. The pH of the solution was adjusted to 2.5 by the addition of 1N HCl. The solution was extracted with DCM (3 × 600 mL), the organic layers combined, dried over anhydrous MgSO₄ and concentrated *in vacuo*. The residue was recrystallized from DCM-ether to give the product which was collected by filtration and dried at 40 °C overnight to yield 38 g (54%) of the octa-acid: ¹³C NMR (D₂O) δ 38.384, 39.704, 51.951, 54.465, 62.934, 67.105, 71.445-74.381 (PEG), 159.772, 173.831, 174.940, 176.359, 176.696.

### Example 14.

**Mel-OMe (20).** Melphalan (**19**, 1.00 g, 3.28mmol) was suspended in 2,2 dimethoxypropane (65.59 mL, 533.49 mmol). To the suspension was added aqueous HCl (36 %, 3.28 mL) and absolute methanol (4 mL). The mixture was warmed to mild reflux with vigorous stirring until solution started to turn slightly brown, followed by stirring at room temperature for 18 hours. The reaction mixture was concentrated *in vacuo* and the crude product precipitated from the residue with ether. The solid was filtered, washed with ether, and purified by silica gel column chromatography (CHCl₃ : MeOH = 9:1, v/v) to yield the desired product (0.47g, 45%): ¹³C NMR δ 39.751, 40.340, 51.912, 53.435, 55.803, 112.124, 126.076, 130.620, 145.033, 175.754.

### Example 15.

**Boc-TML1β-Mel-OMe (22).** EDC (0.52 g, 2.70 mmol) and DMAP (0.988 g, 8.10 mmol) were added to a mixture of **21** (0.531 g, 1.35 mmol) and 20 (0.863 g, 2.70 mmol) in anhydrous DCM (15 mL) and anhydrous DMF (5 mL) at 0 °C in an ice bath.. The reaction mixture was stirred at room temperature overnight under nitrogen then concentrated *in vacuo*. The residue was redissolved in DCM (75 mL) and washed three times with 25mL 1N HCl. The organic layer was dried over anhydrous magnesium sulfate, concentrated, and purified by silica gel column chromatography (ethyl acetate:hexane = 7:3, v/v) to yield the desired product (0.757 g, 80.8 %): ¹³C NMR δ 20.120, 25.306, 28.294, 31.768, 35.427, 35.947, 36.669, 39.505, 40.311, 49.324, 51.959, 53.234, 53.453, 79.467, 112.095, 123.374, 125.169, 130.439, 132.856, 133.427, 136.666, 138.697, 145.091, 149.841, 156.081, 170.888, 172.298.

### Example 16.

**TML1β-Mel-OMe TFA Salt (23).** Compound 22 (0.757 g, 1.09 mmol) was stirred in DCM (5mL) and TFA (2.5 mL) at room temperature for 2 hours. The reaction solution was concentrated, redissolved in minimal DCM, and precipitated with ether. The product was collected by filtration to yield the desired product (0.222g, 35.9 %): ¹³C NMR (CDCl₃ + CD₃OD) δ 20.026, 25.146, 31.738, 31.892, 35.271, 36.219, 39.163, 40.340, 49.006, 52.219, 53.396, 112.073, 123.260, 124.756, 130.377, 133.026, 133.180, 136.815, 138.595, 145.110, 149.283, 171.069, 171.619, 172.630.

### Example 17.

**PEG-cmc-TML1β-Mel-OMe (24).** A mixture of PEG-cmc-Asp-Asp-OH (**12**, 1.6g, 0.0391mmol), 23 (0.277g, 0.391mmol), EDC (0.076g, 0.391mmol), and DMAP (0.155g, 1.269mmol) in anhydrous DCM (23 mL) and anhydrous DMF (6 mL) was stirred overnight at room temperature under nitrogen. The solution was concentrated *in vacuo* and the residue recrystallized from 130mL IPA to yield the product (1.543g, 92.5 %). The amount of melphalan in the product measured by UV assay was 2.86% wt/wt: ¹³C NMR δ 19.642, 24.788, 31.175, 34.350, 35.975, 38.817, 39.905, 48.558, 51.553, 52.808, 60.897, 62.331, 65.145-72.878 (PEG), 111.394, 122.761, 124.425, 129.698, 132.105, 132.878, 135.804, 137.737, 144.316, 149.065, 160.432, 170.608, 171.598.

### Example 18.

**Boc-TML1β-AraC (25).** A solution of Ara-C (1, 9.88 g, 40.66 mmol) in anhydrous pyridine (85 mL) was added to a mixture of 21 (4.0 g, 10.17 mmol), HOBT (5.49 g, 40.66 mmol), EDC (15.61 g, 81.32 mmol), and NMM (8.93mL, 8.21g, 81.32mmol, 8eq) in anhydrous pyridine (200 mL). The reaction mixture was stirred for 48 hours at 40 °C under nitrogen, followed by concentration *in vacuo*. The residue was redissolved in DCM (300 mL), washed three times with water (100 mL) and twice with 0.1N HCl (100 mL). The organic layer was dried over magnesium sulfate, concentrated, and purified by silica gel column chromatography (CHCl₃ - MEOH = 9:1, v/v) to yield the desired product (3.26 g, 52%): ¹³C NMR δ 20.315, 25.560, 28.522, 31.660, 35.520, 36.200, 39.221, 50.239, 61.719, 75.171, 76.698, 79.635, 85.341, 88.052, 96.435, 122.894, 132.519, 133.190, 136.186, 138.007, 146.222, 149.109, 155.906, 162.191, 171.733.

### Example 19.

**TML1β-AraC TFA salt (26).** Compound **25** (3 g, 4.85 mmol) was dissolved in DCM (15 mL) followed by addition of TFA (7.5 mL) at 0 °C. Reaction mixture was stirred at 0 °C for 1.2 hours and concentrated *in vacuo* in a cool water bath. Residue was precipitated with DCM-ether to yield the desire product (2.37 g, 77 %): ¹³C NMR (CDCl₃ + CD₃OD) δ 20.0, 25.3, 31.5, 31.7, 35.0, 38.9, 50.2, 60.9, 75.1, 75.8, 85.7, 88.1, 94.9, 109.7, 113.5, 117.3, 121.1, 122.5, 132.6, 136.4, 138.4, 148.7, 149.5, 150.1, 159.2, 159.6, 160.1, 160.6, 161.1, 170.6, 172.7

### Example 20.

**PEG-cmc-Asp-Asp-TML1β-AraC, octamer (27).** Compounds **26** and **18** were subjected to the same condition as in Example 18 to prepare **27.**

### Example 21.

In vitro and in vivo data for compounds 6a and **6b.**

In this Example, in vivo and in vitro data are presented and compared to unmodified Ara-C.

### In Vivo

Athymic nude mice were implanted subcutaneous with a 4-5 mm³ tissue fragment of LX-1 collected from donor mice. The tumor trocar site was observed twice weekly and measured once palpable. The tumor volume for each mouse was determined by measuring two dimensions with calipers and calculated using the formula: tumor volume = (length x width²)/2. When tumors reached the average volume of 90 mm³, the mice were divided into their experimental groups which consisted of unmodified Ara-C and PEG-Ara-C compounds. The mice were sorted to evenly distribute tumor size, grouped into 4 to 6 mice/group, and ear punched for permanent identification. Drugs were administered intravenously q3d x 4 (Day 1, 4, 7 and 10) via the tail vein at an approximate rate of 0.5 mL per minute. Compounds were given both at an equal molar basis (absolute amount of active) of 20 mg/kg and at close their respective MTD (Ara-C, 100 mg/kg/dose (toxicity); 6a and 6b, 40 mg/kg/dose (volume). Mouse weight and tumor size were measured at the beginning of study and twice weekly through week 4. Drug effectiveness was determined by comparing tumor growth in treated versus untreated (no vehicle) control mice. Five types of endpoints were used as the basis for comparison: (a) mean tumor volumes at Day 28; (b) mean percent change in individual tumor volumes from initial; (c) percent difference in tumor volume (%T/C), measured when the control group's median tumor volume reached approximately 800 - 1100 mm³ (exponential growth phase); (d) percent difference in tumor volume (%T/C) at Day 21 (∼2000 mm³) and (e) the number of tumor regression (smaller tumor volume on Day 28 compared to Day 1) per group.

### Results

Compound 6b demonstrated better antitumor activity than native Ara-C at only 20% of the active parent compound's dose. Compound 6a also demonstrated significant efficacy. Although the %T/C was about twice of that which was recorded for 6b, it nonetheless compared vary favorably against native Ara-C, especially considering that the inventive compound was given at only 20% of the active parent compound's dose.

| **Compound** | ***t*_{1/2} (h)*^{a}* Rat Plasma** | **IC₅₀ (nM)*^{a}* P388/O** | **LX-1 %T/C*^{b}*** |
|---|---|---|---|
| Ara-C | - | 10 | 74.0 |
| | | | (100 mg/kg) |
| Compound 6a | 2.1 | 123 | 122 |
| | | | (20 mg/kg) |
| Compound 6b | 53 | 958 | 59.3 |
| | | | (20 mg / kg) |

| | | | |
|---|---|---|---|
| *^{a}* All experiments were done at 37 °C in duplicate and *t*_{1/2} was measured by the disappearance of PEG derivatives. Standard deviation of measurements = ± 10 %. *^{b}* Mean baseline tumor volume was 1000 mm³. | | | |

### IN VITRO BIOASSAY

A series of in vitro assays were conducted to determine the IC₅₀ for unmodified Ara-C and compound 10 using the P388/O (murine lymphoid neoplasm, Southern Research Institute) cell line. The P388/0 cells were grown in RPMI 1640 medium (Whittaker Bioproducts, Walkersville, Maryland) + 10% FBS (Hyclone Inc., Logan UT). Bioassays were performed in their respective media containing antibiotics and fungizone.

Ara-C was dissolved in DMSO and diluted to the appropriate concentration in culture media. The PEG-Ara-C compounds were dissolved in water and diluted to the appropriate concentrations in culture media.

The assays were performed in duplicate in 96-well microtiter cell culture plates. Two fold serial dilution of the compounds were done in the microtiter plates. Cells were detached by incubating with 0.1% Trypsin/Versene at 37°. Trypsin was inactivated by adding the appropriate media for each cell line containing 10% FBS. To each well of the microtiter plates, 10,000 cells were added. After three days, cell growth was measured by addition of a metabolic indicator dye, Alamar Blue, according to the manufacturer's protocol. The IC₅₀ value for the test compounds and reference compound are provided above in the Table.

While there have been described what are presently believed to be the preferred embodiments of the invention, those skilled in the art will realize that changes and modifications may be made without departing from the spirit of the invention. It is intended to claim all such changes and modifications as fall within the true scope of the invention.

## Claims

1. A compound comprising the formula: 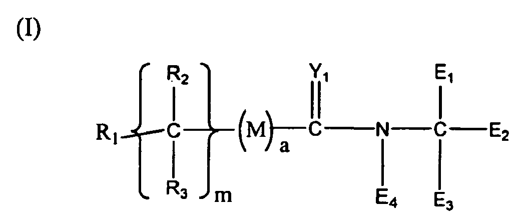 wherein:
R₁ is a polymeric residue;
Y₁ is O, S or NR₄;
M is O, S or NR₅;
E₁ is 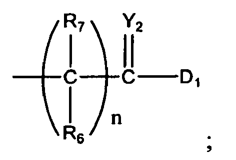
E₂ is E₁ or 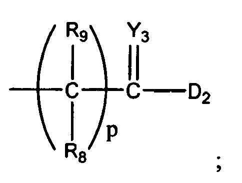
E₃₋₄ are independently H, E₁ or 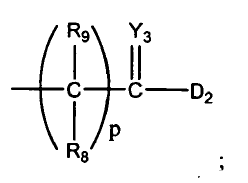
(a) is zero or one;
(m) is zero or a positive integer;
(n) and (p) are independently 0 or a positive integer;
Y₂₋₃ are independently O, S or NR₁₀;
R₂₋₁₀ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁-₆ heteroalkyls, substituted C₁₋₆ heteroalkyls, C₁₋₆ alkoxy, phenoxy and C₁₋₆ heteroalkoxy;
D₁ and D₂ are independently 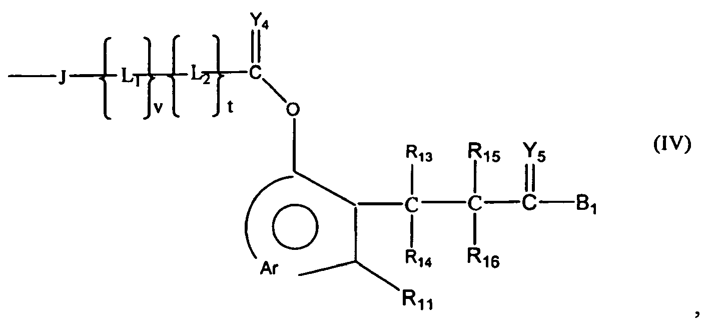 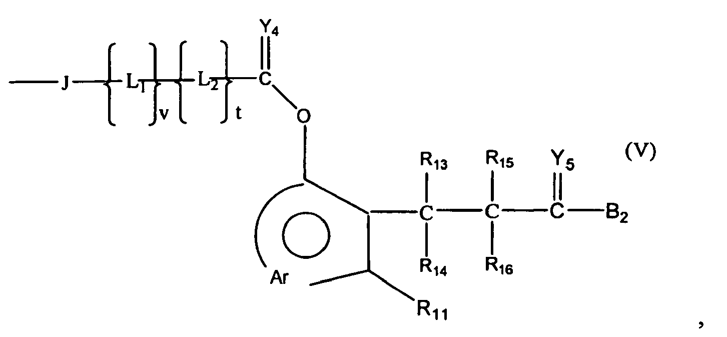
or a terminal branching group;
wherein (v) and (t) are independently 0 or a positive integer up to about 6;
J is NR₁₂ or 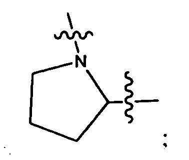 L₁ and L₂ are independently selected bifuntional linkers;
Y₄₋₇ are independently selected from the group consisting of O, S and NR₁₄;
R₁₁₋₁₄ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls; aryls, substituted aryls, aralkyls, C₁₋₆ heteroalkyls, substituted C₁₋₆ heteroalkyls,
C₁₋₆ alkoxy, phenoxy and C₁₋₆ heteroalkoxy;
Ar is a moiety which when included in Formula (I) forms a multi-substituted aromatic hydrocarbon or a multi-substituted heterocyclic group;
B₁ and B₂ are independently selected from the group consisting of leaving groups, OH, residues of hydroxyl-containing moieties or amine-containing moieties;
said terminal branching group comprises the formula 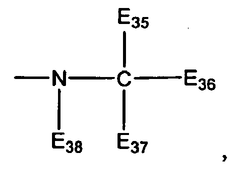 wherein
E₃₅ is 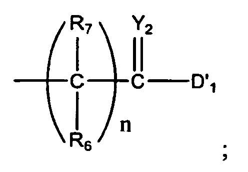 E₃₆₋₃₈ are independently H, E₃₅ or 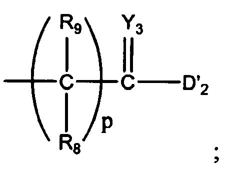 D'₁ is 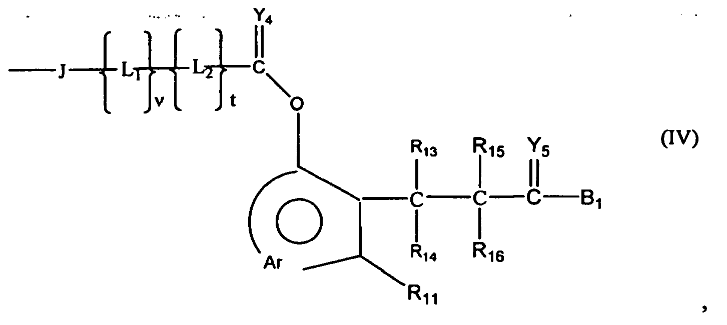 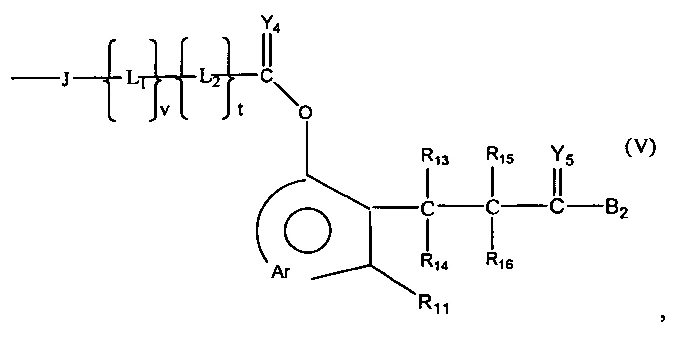 or 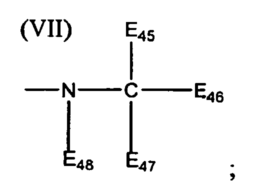 D'₂ is OH, 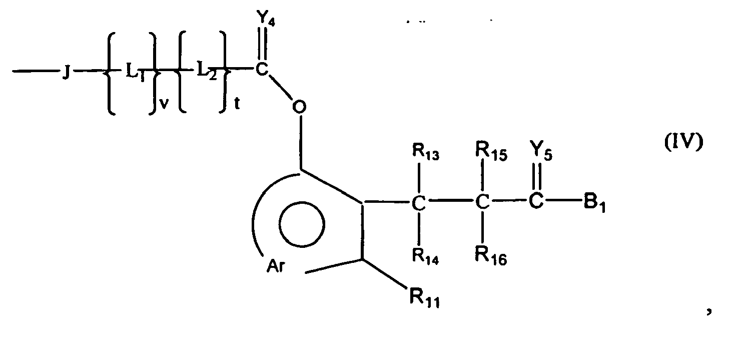 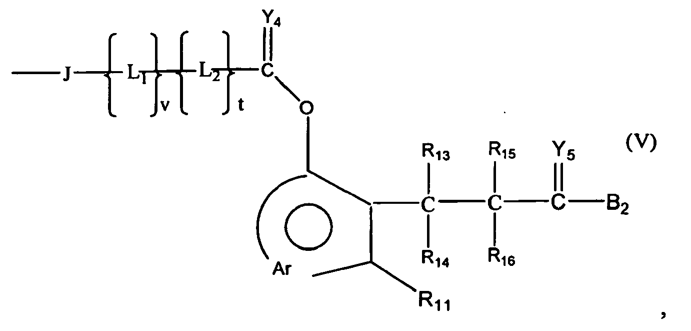 or 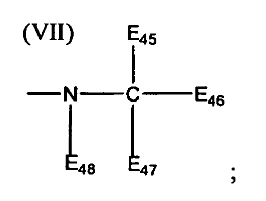 wherein
E₄₅ is 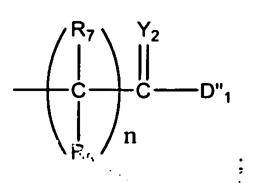 E₄₆₋₄₈ are independently H, E₄₅ or 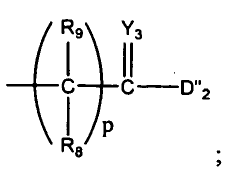 wherein
D"₁ is 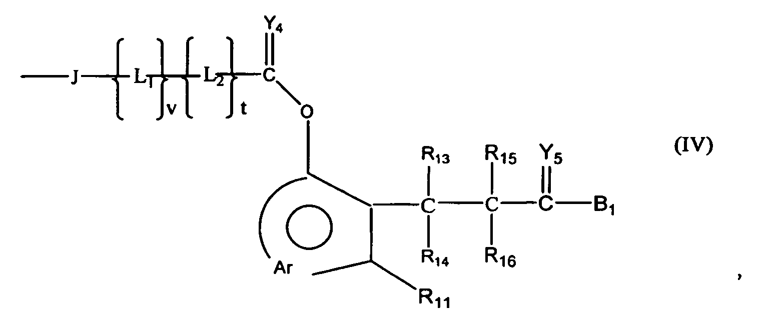 , or 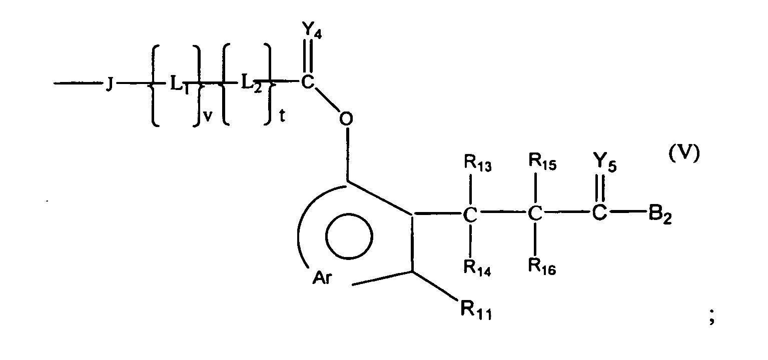 D"₂ is OH, 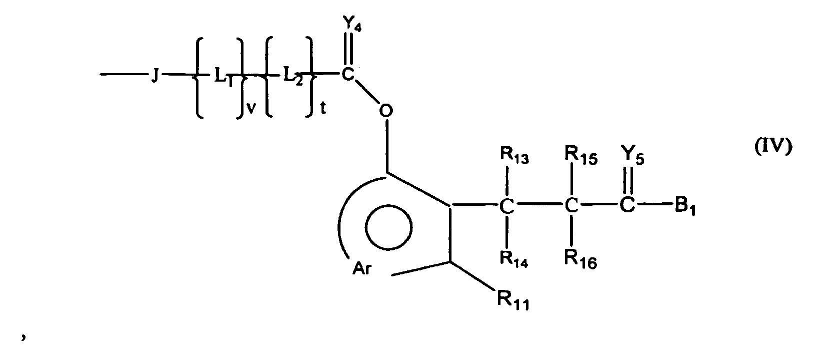 or

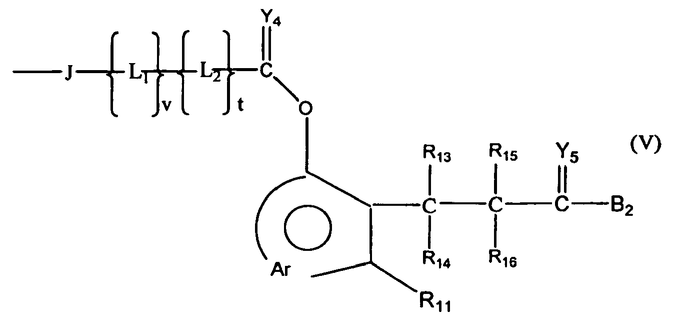

2. The compound of claim 1, wherein R₁ further comprises a capping group A, selected from the group consisting of hydrogen, NH_{2,} OH, CO₂H, C₁₋₆ moieties and

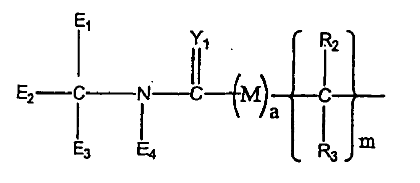

3. A compound of claim 2, comprising the formula:

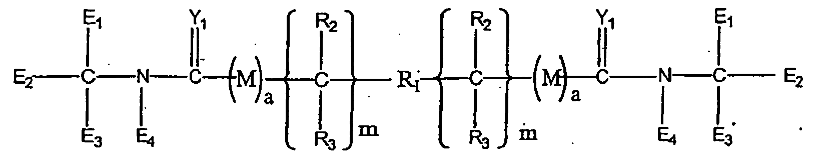

4. The compound of claim 3, wherein Y₁ is O.

5. The compound of claim 1, wherein R₁ comprises a polyalkylene oxide residue.

6. The compound of claim 5, wherein R₁ comprises a polyethylene glycol residue.

7. The compound of claim 3, wherein R₁ comprises a polyethylene glycol residue.

8. The compound of claim 5; wherein R₁ is selected from the group consisting of
-C(=Y₆)-(CH₂)_{f}-O-(CH₂CH₂O)ₓ-A,
-C(=Y₆)-Y₇-(CH₂)_{f}-O-(CH₂CH₂O)ₓ-A,
-C(=Y₆)-NR₂₃-(CH₂)_{f}-O-(CH₂CH₂O)ₓ-A,
-(CR₂₄R₂₅)ₒ-O-(CH₂)_{f}O-(CH₂CH₂O)ₓ-A,
-NR₂₃-(CH₂)_{f}-O-(CH₂CH₂O)ₓ-A,
-C(=Y₆)-(CH₂)_{f}-O-(CH₂CH₂O)ₓ-(CH₂)_{f}-C(=Y₆)-,
-C(=Y₆)-Y7-(CH₂)_{f}-O-(CH₂CH₂O)ₓ-(CH₂)_{f}-Y₇-C(=Y₆)-,
-C(=Y₆)-NR₂₃-(CH₂)_{f}-O(CH₂CH₂O)ₓ-(CH₂)_{f}-NR₂₃-C(=Y₆)-,
-(CR₂₄R₂₅)ₒ-O-(CH₂)_{f}-O-(CH₂CH₂O)ₓ-(CH₂)_{f}-O-(CR₂₄R₂₅)ₒ-, and
-NR₂₃-(CH₂)_{f}-O-(CH₂CH₂O)ₓ-(CH₂)_{f}-NR₂₃-
wherein: Y₆ and Y₇ are independently O, S or NR₂₃;
x is the degree of polymerization;
R_{23,} R₂₄ and R₂₅ are independently selected from among H, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
*e* and *f* are independently zero, one or two; and
A is a capping group.

9. The compound of claim 8, wherein R₁ comprises -O-(CH₂CH₂O)ₓ and x is a positive integer so that the weight average molecular weight is at least about 20,000.

10. The compound of claim 3, wherein R₁ has a weight average molecular weight of from about 20,000 to about 100,000.

11. The compound of claim 3, wherein R₁ has a weight average molecular weight of from about 25,000 to about 60,000.

12. A compound of claim 3, comprising the formula

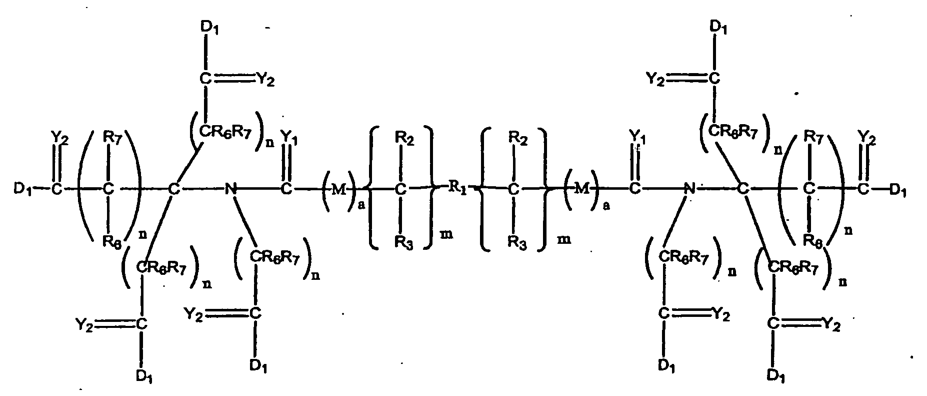

13. The compound of claim 12, wherein D₁ is

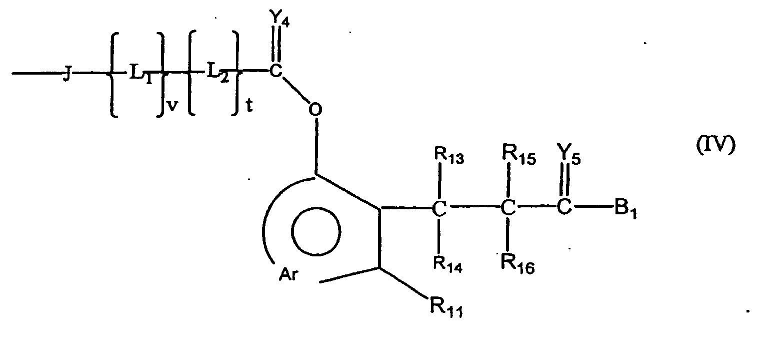

14. The compound of claim 12, wherein D₁ is

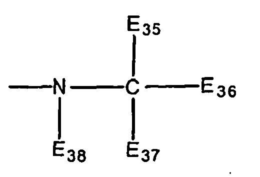

15. The compound of claim 1, wherein L₁ is (CH₂CH₂O)₂.

16. The compound of claim 1, wherein L₂ is selected from the group consisting of -CH₂-, -CH(CH₃)-, -CH₂ C(O)NHCH(CH₃)-, -(CH₂)₂-, -CH₂C(O)NHCH₂-, -(CH₂)₂-NH-, -(CH₂)₂-NH-C(O)(CH₂)₂NH- and -CH₂C(O)NHCH(CH₂CH(CH₃)₂)-.

17. A compound of claim 1, selected from the group consisting of: 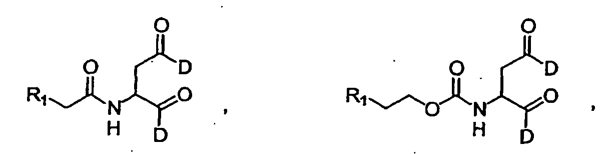 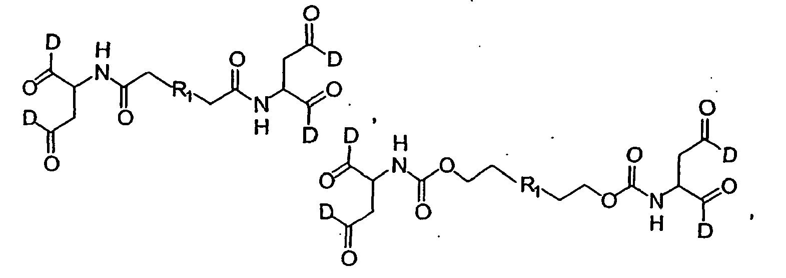 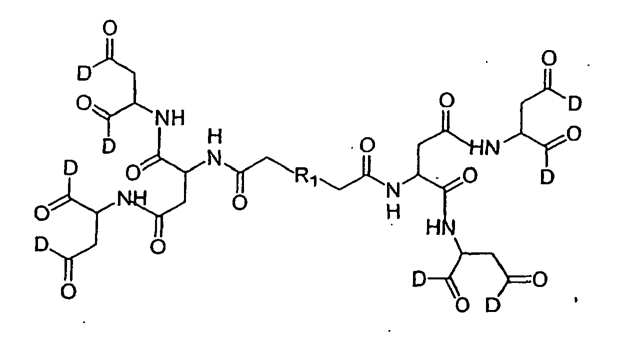 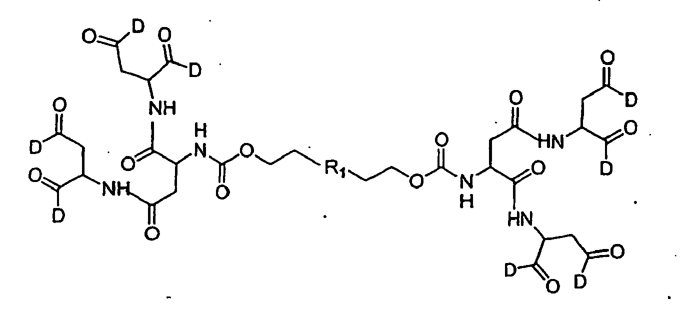 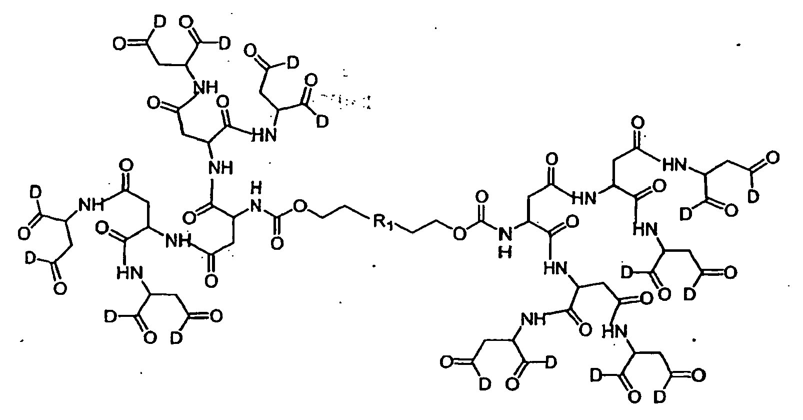 and 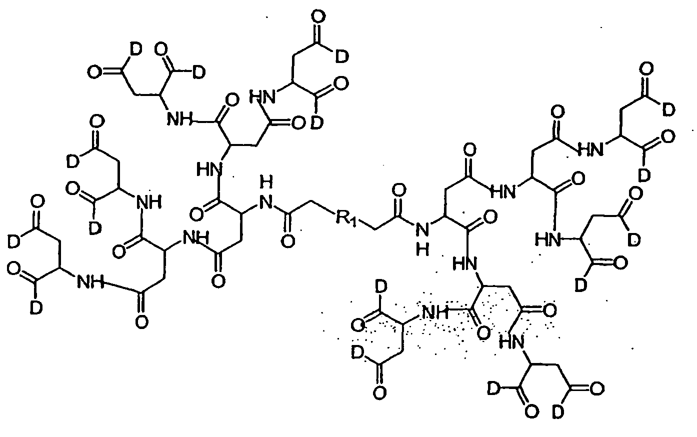 wherein R₁ is a PEG residue and D is selected from the group consisting of: 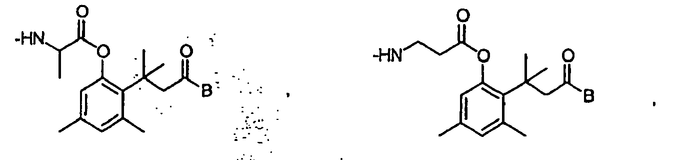 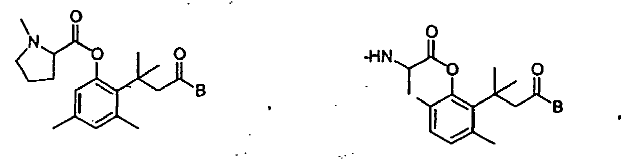 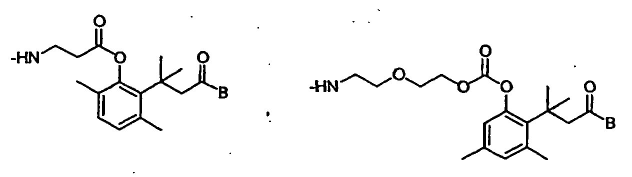 and 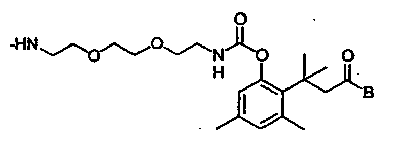 where B is a residue of an amine or a hydroxyl- containing drug.

18. A compound of claim 17, wherein B is a residue of a member of the group consisting of: daunorubicin, doxorubicin; *p*-aminoaniline mustard, melphalan, Ara-C (cytosine arabinoside), leucine-Ara-C, and gemcitabine

19. A pharmaceutical composition comprising a prodrug compound as defined in any one of claims 1-18 and a pharmaceutically acceptable carrier.

20. A compound as defined in any one of claims 1-18 for use in a method of treatment of the human or animal body by therapy.

21. The compound of claim 1, wherein Ar comprises the formula: 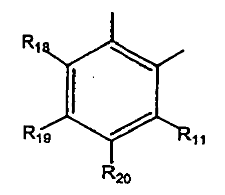 wherein R₁₁ and R₁₈₋₂₀ are individually selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroakoxy.

22. The compound of claim 21, wherein R₁₁ and R₁₈₋₂₀ are each H or CH₃.

23. A method of preparing a polymer conjugate, comprising:
reacting a compound of the formula (VIII): 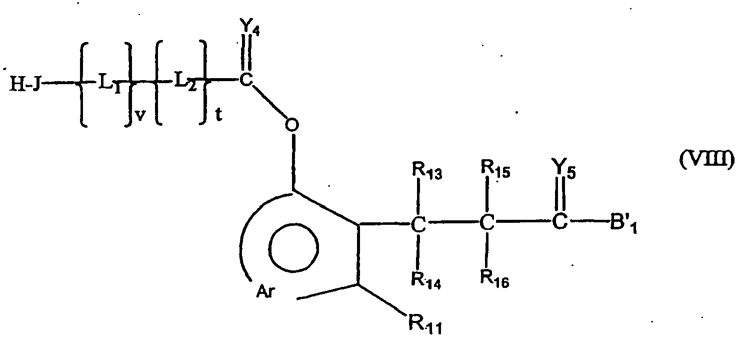
wherein
(v) and (t) are independently 0 or a positive integer up to about 6;
J is NR₁₂ or 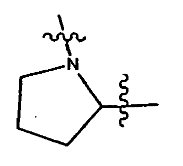 L₁ and L₂ are independently selected bifunctional linkers;
Y₄₋₅ are independently selected from the group consisting of O, S and NR₁₇;
R₁₁₋₁₇ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
Ar is a moiety which when included in Formula (I) forms a multi-substituted aromatic hydrocarbon or a multi-substituted heterocyclic group; and
B'₁ is a residue of a hydroxyl- or an amine-containing moiety;
with a compound of the formula (IX): 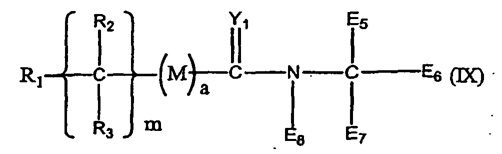 wherein
E₅ is 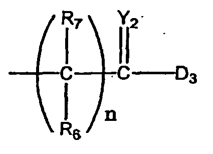 E₆₋₈ are independently H, E₅ or 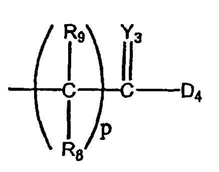 but E₆₋₈ are not all H;
D₃ and D₄ are independently OH, a leaving group which is capable of reacting with an unprotected amine or hydroxyl or a terminal branching group as defined in claim 1, but and D₃ and D₄ are not both OH;
R₁ is a polymeric residue;
Y₁ is O, S or NR₄;
M is O, S or NR₅;
(a) is zero or one;
(m) is 0 or a positive integer;
(n) and (p) are independently 0 or a positive integer;
Y₂₋₃ are independently O, S or NR₁₀; and
R₂₋₁₀ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
under conditions sufficient to cause a polymeric conjugate to be formed.

## Patentansprüche

1. Verbindung, umfassend die Formel: 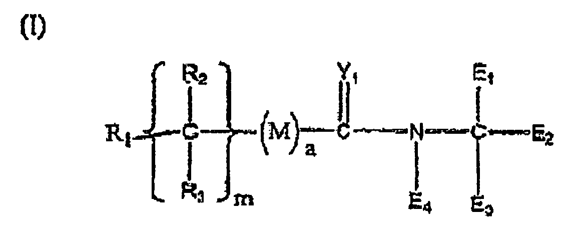 wobei:
R₁ ein polymerer Rest ist;
Y₁ O, S oder NR₄ ist;
M O, S oder NR₅ ist;
E₁ 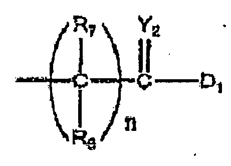 ist;
E₂ E₁ oder 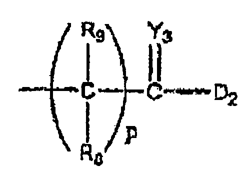 ist;
E₃₋₄ unabhängig voneinander H, E₁ oder 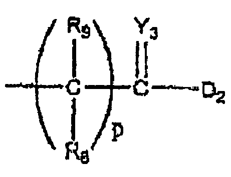 sind;
(a) null oder eins ist;
(m) null oder eine positive ganze Zahl ist;
(n) und (p) unabhängig voneinander 0 oder eine positive ganze Zahl sind;
Y₂₋₃ unabhängig voneinander O, S oder NR₁₀ sind;
R₂₋₁₀ unabhängig voneinander ausgewählt sind aus der Gruppe, bestehend aus Wasserstoff, C₁₋₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxy und C₁₋₆-Heteroalkoxy;
D₁ und D₂ unabhängig voneinander 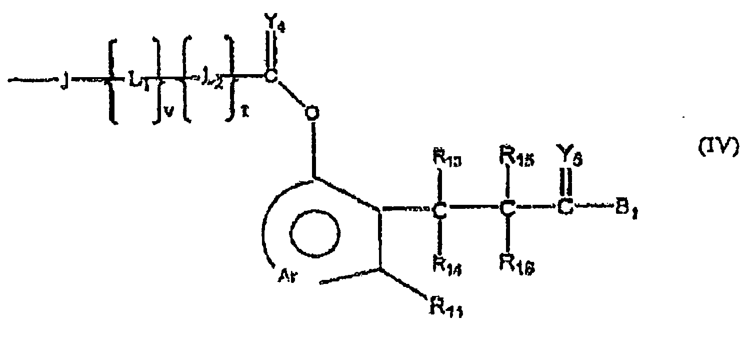 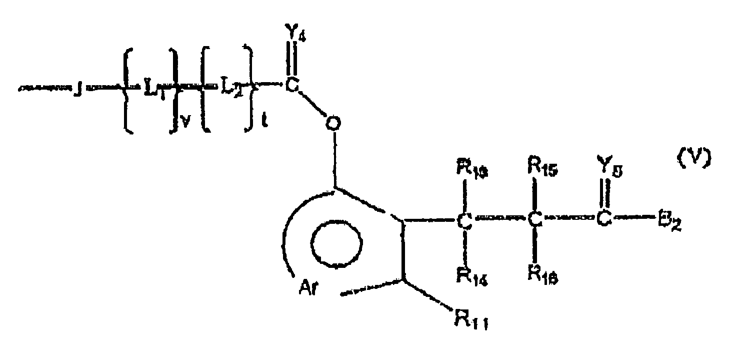
oder eine endständige Verzweigungsgruppe sind;
wobei (v) und (t) unabhängig voneinander 0 oder eine positive ganze Zahl von bis zu etwa 6 sind;
J NR₁₂ oder 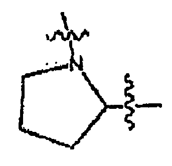 ist;
L₁ und L₂ unabhängig voneinander ausgewählt sind aus einer bifunktionellen Verbindungsgruppe;
Y₄₋₇ unabhängig voneinander ausgewählt sind aus der Gruppe, bestehend aus O, S und NR₁₄;
R₁₁₋₁₄ unabhängig voneinander ausgewählt sind aus der Gruppe, bestehend aus Wasserstoff, C₁₋₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxy und C₁₋₆-Heteroalkoxy;
Ar eine Einheit ist, die, falls in Formel (I) eingeschlossen, einen mehrfach substituierten aromatischen Kohlenwasserstoff oder eine mehrfach substituierte heterocyclische Gruppe bildet;
B₁ und B₂ unabhängig voneinander ausgewählt sind aus der Gruppe, bestehend aus Abgangsgruppen, OH, Resten von hydroxyhaltigen Einheiten oder aminhaltigen Einheiten;
wobei die endständige Verzweigungsgruppe die Formel 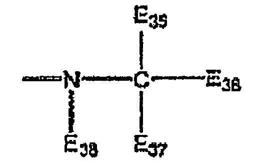 umfasst,
wobei
E₃₅ 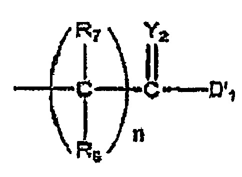 ist;
E₃₆₋₃₈ unabhängig voneinander H, E₃₅ oder 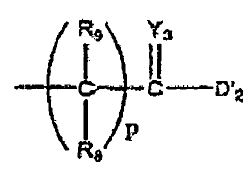 sind;
D'₁ 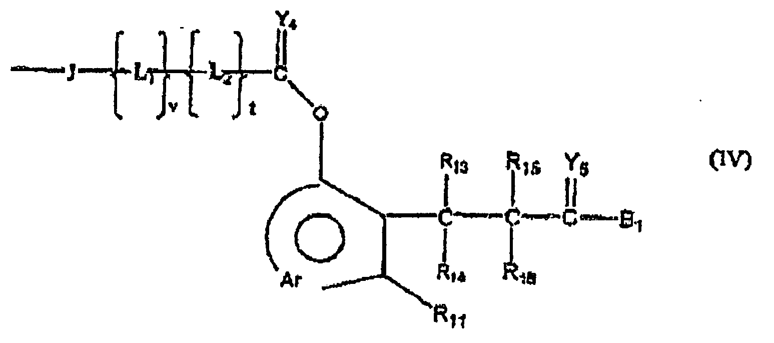 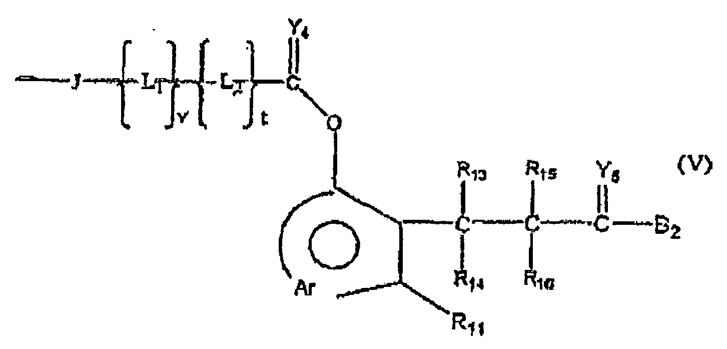 oder 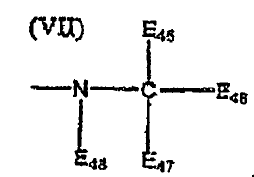 ist;
D'₂ OH, 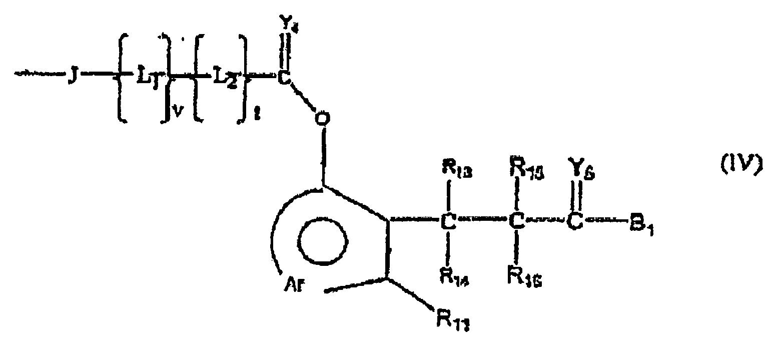 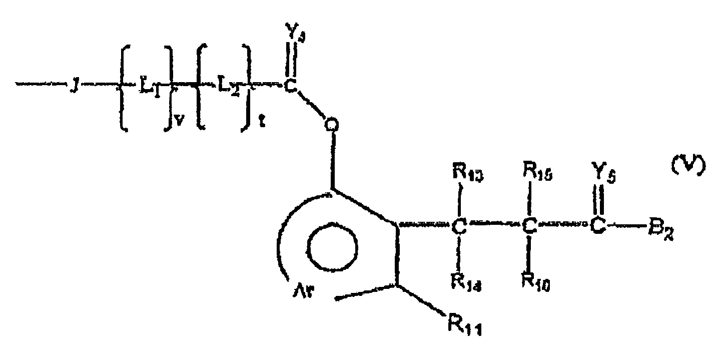 oder 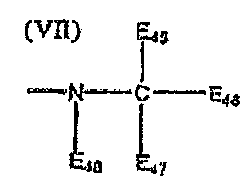 ist;
wobei
E₄₅ 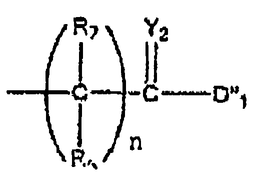 ist;
E₄₆₋₄₈ unabhängig voneinander H, E₄₅ oder 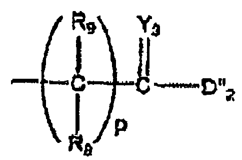 sind;
wobei D"₁ 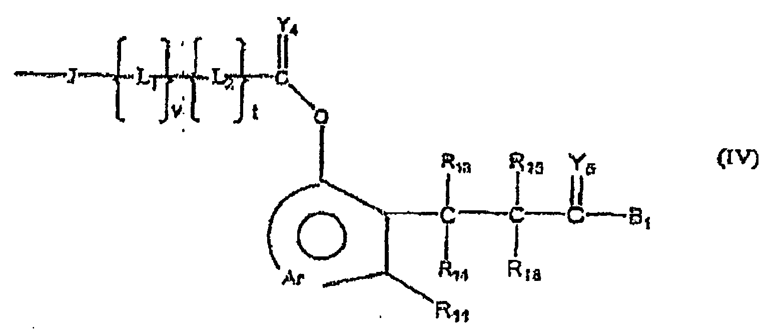 oder 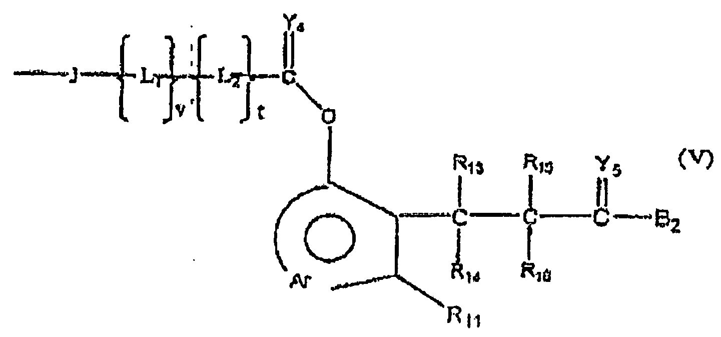 ist;
D"₂ OH, 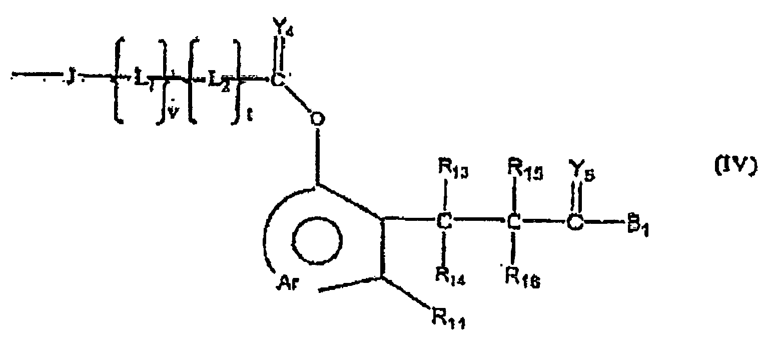 oder 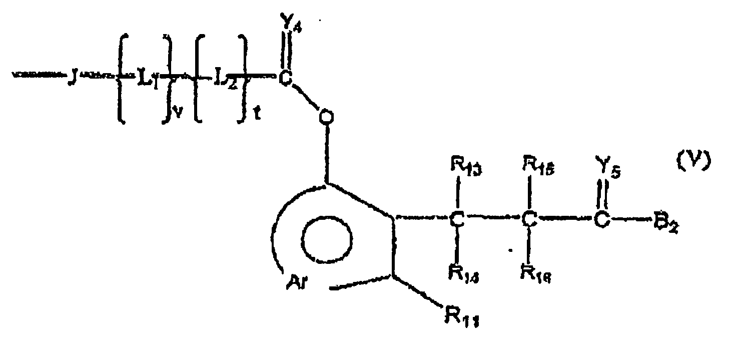 ist.

2. Verbindung nach Anspruch 1, wobei R₁ ferner eine endständige Gruppe A umfasst, ausgewählt aus der Gruppe, bestehend aus Wasserstoff, NH₂, OH, CO₂H, C₁₋₆-Einheiten und

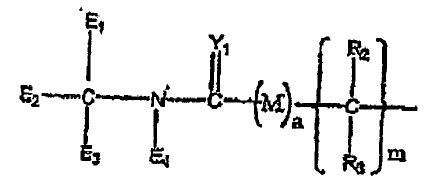

3. Verbindung nach Anspruch 2, umfassend die Formel

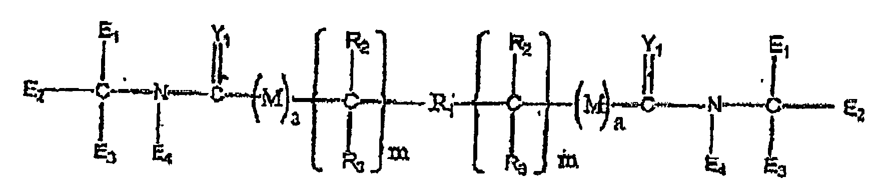

4. Verbindung nach Anspruch 3, wobei Y₁ O ist.

5. Verbindung nach Anspruch 1, wobei R₁ einen Polyalkylenoxidrest umfasst.

6. Verbindung nach Anspruch 5, wobei R₁ einen Polyethylenglycolrest umfasst.

7. Verbindung nach Anspruch 3, wobei R₁ einen Polyethylengrlycolrest umfasst.

8. Verbindung nach Anspruch 5, wobei R₁ ausgewählt ist aus der Gruppe, bestehend aus
-C(=Y₆)-(CH₂)_{f}-O-(CH₂CH₂O)ₓ-A,
-C(=Y₆)-Y₇-(CH₂)_{f}-O-(CH₂CH₂O)ₓ-A,
-C(=Y₆)-NR₂₃-(CH₂)_{f}-O-(CH₂CH₂O)ₓ-A,
-(CR₂₄R₂₅)ₒ-O-(CH₂)_{f}-O-(CH₂CH₂O)ₓ-A,
-NR₂₃-(CH₂)_{f}-O-(CH₂CH₂O)ₓ-A,
-C(=Y₆)-(CH₂)_{f}-O-(CH₂CH₂O)ₓ-(CH₂)_{f}-C(=Y₆)-,
-C(=Y₆)-Y₇-(CH₂)_{f}-O-(CH₂CH₂O)ₓ-(CH₂)_{f}-Y₇-C(=Y₆)-,
-C(=Y₆)-NR₂₃-(CH₂)_{f}-O-(CH₂CH₂O)ₓ-(CH₂)_{f}-NR₂₃-C(=Y₆)-,
-(CR₂₄R₂₅)ₒ-O-(CH₂)_{f}-O-(CH₂CH₂O)ₓ-(CH₂)_{f}-O-(CR₂₄R₂₅)_{o-,} und
-NR₂₃-(CH₂)_{f}-O-(CH₂CH₂O)ₓ-(CH₂)_{f}-NR₂₃-
wobei Y₆ und Y₇ unabhängig voneinander O, S oder NR₂₃ sind;
x der Polymerisationsgrad ist;
R₂₃, R₂₄ und R₂₅ unabhängig voneinander ausgewählt sind aus H, C₁-₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxyund C₁₋₆-Heteroalkoxy;
e und f unabhängig voneinander null, eins oder zwei sind; und
A eine endständige Gruppe ist.

9. Verbindung nach Anspruch 8, wobei R₁ -O-(CH₂CH₂O)ₓ umfasst und x eine derartige positive ganze Zahl ist, dass das Gewichtsmittel des Molekulargewichts mindestens etwa 20.000 beträgt.

10. Verbindung nach Anspruch 3, wobei R₁ ein Gewichtsmittel des Molekulargewichts von etwa 20.000 bis etwa 100.000 aufweist.

11. Verbindung nach Anspruch 3, wobei R₁ ein Gewichtsmittel des Molekulargewichts von etwa 25.000 bis etwa 60.000 aufweist.

12. Verbindung nach Anspruch 3, umfassend die Formel

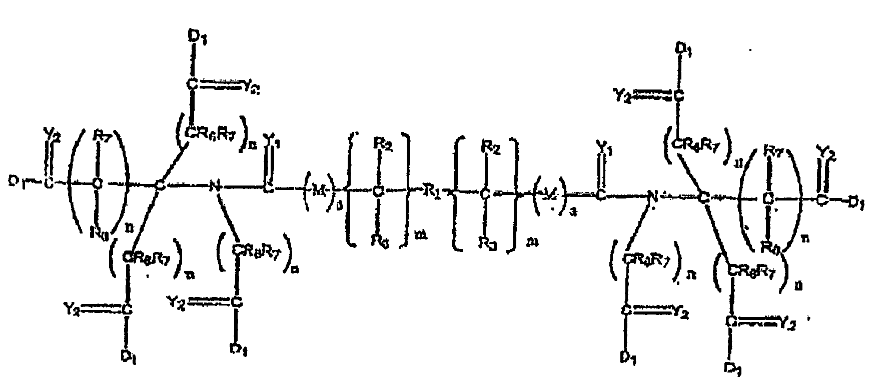

13. Verbindung nach Anspruch 12, wobei D₁ 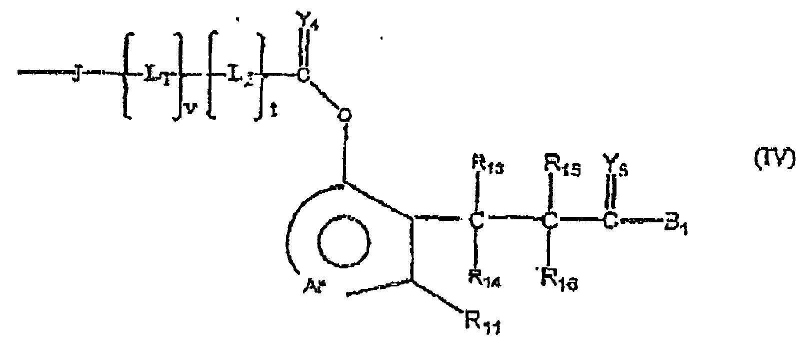 ist.

14. Verbindung nach Anspruch 12, wobei D₁ 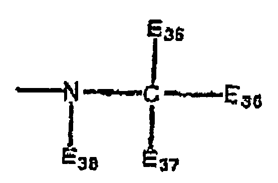 ist.

15. Verbindung nach Anspruch 1, wobei L₁ (CH₂CH₂O)₂ ist.

16. Verbindung nach Anspruch 1, wobei L₂ ausgewählt ist aus der Gruppe, bestehend aus
-CH₂-, CH(CH₃)-, -CH₂C(O)NHCH(CH₃)-, -(CH₂)₂-, -CH₂C(O)NHCH₂-, - (CH₂)₂-NH-, -(CH₂)₂-NH-C(O)(CH₂)₂NH- und - CH₂C(O)NHCH(CH₂CH(CH₃)₂-.

17. Verbindung nach Anspruch 1, ausgewählt aus der Gruppe, bestehend aus 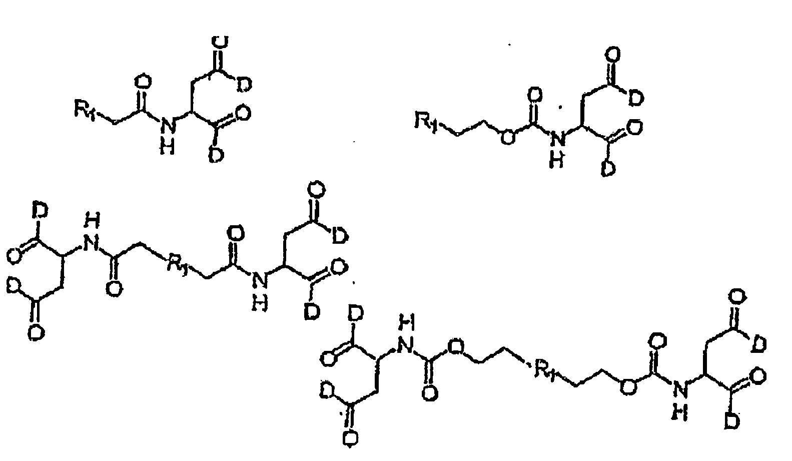 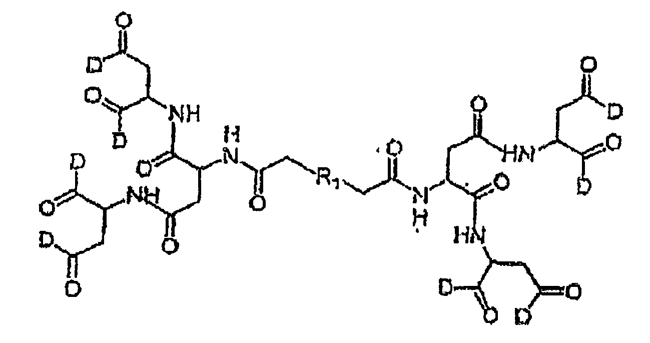 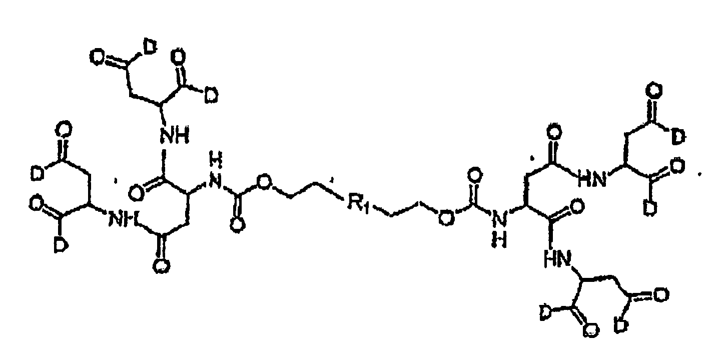 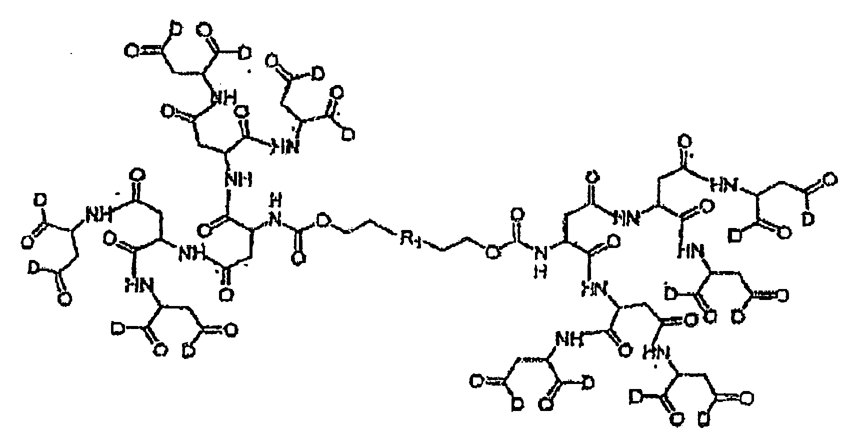 und 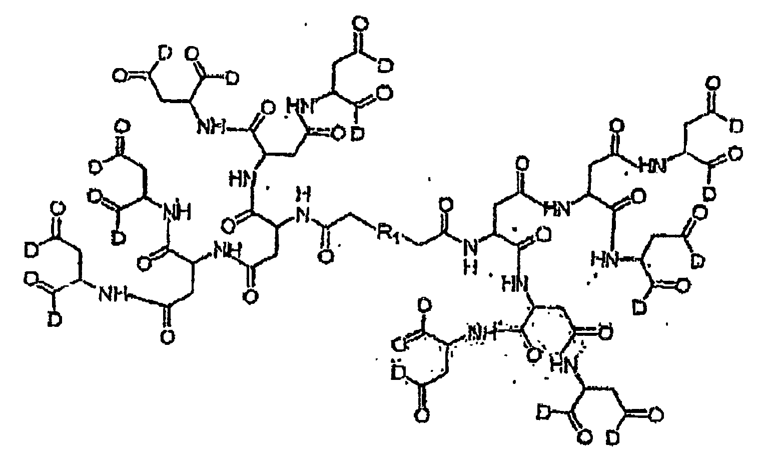 wobei R₁ ein PEG-Rest ist und D ausgewählt ist aus der Gruppe, bestehend aus 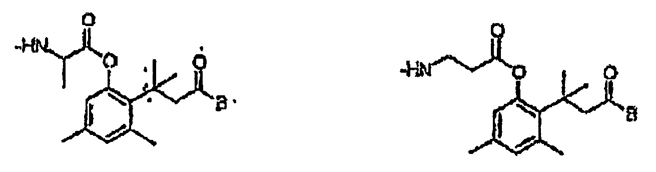 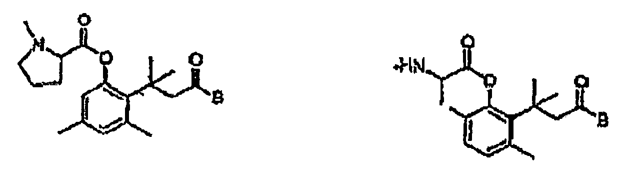 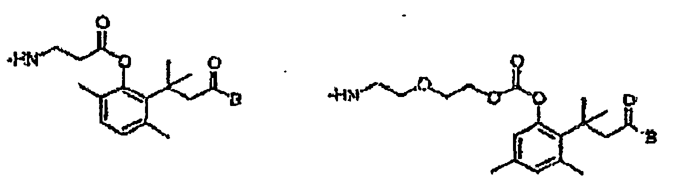 und 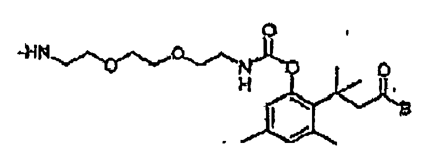 wobei B ein Rest eines amin- oder hydroxyhaltigen Arzneistoffs ist.

18. Verbindung nach Anspruch 17, wobei B ein Rest eines Vertreters der Gruppe ist, bestehend aus Daunorubicin, Doxorubicin; p-Aminoanilinsenf, Melphalan, Ara-C (Cysteinarabinosid), Leucin-Ara-C und Gencitabin.

19. Arzneimittel, umfassend eine wie in einem der Ansprüche 1-18 definierte Prodrug-Verbindung und einen pharmazeutisch verträglichen träger.

20. Verbindung, wie in einem der Ansprüche 1-18 definiert, zur Verwendung in einem Verfahren zur therapeutischen Behandlung des menschlichen oder tierischen Körpers.

21. Verbindung nach Anspruch 1, wobei Ar die Formel 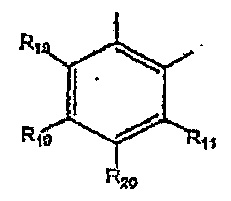 umfasst,
wobei R₁₁ und R₁₈₋₂₀ einzeln ausgewählt sind aus der Gruppe, bestehend aus Wasserstoff, C₁₋₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxy und C₁₋₆-Heteroalkoxy.

22. Verbindung nach Anspruch 21, wobei R₁₁ und R₁₈₋₂₀ jeweils H oder CH₃ sind.

23. Verfahren zur Herstellung eines Polymerkonjugats, umfassend Umsetzen einer Verbindung der Formel (VIII) 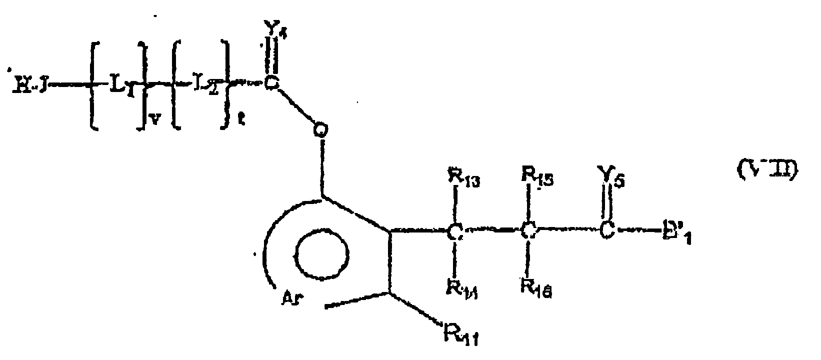 wobei
(v) und (t) unabhängig voneinander 0 oder eine positive ganze Zahl von bis zu etwa 6 sind;
J NR₁₂ oder 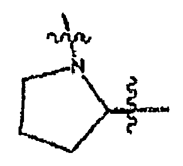 ist;
L₁ und L₂ unabhängig voneinander ausgewählt sind aus bifunktionellen Verbindungsgruppen;
Y₄₋₅ unabhängig ausgewählt sind aus der Gruppe, bestehend aus O, S und NR₁₇;
R₁₁₋₁₇ unabhängig voneinander ausgewählt sind aus der Gruppe, bestehend aus Wasserstoff, C₁₋₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxy und C₁₋₆-Heteroalkoxy;
Ar eine Einheit ist, die, falls in Formel (I) eingeschlossen, einen mehrfach substituierten aromatischen Kohlenwasserstoff oder eine mehrfach substituierte heterocyclische Gruppe bildet; und
B'₁ ein Rest einer hydroxy- oder aminhaltigen Einheit ist;
mit einer Verbindung der Formel (IX) 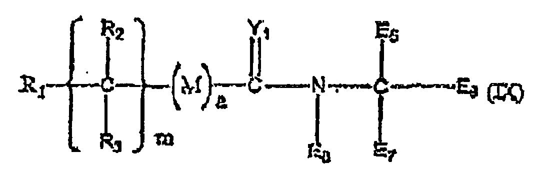 wobei
E₅ 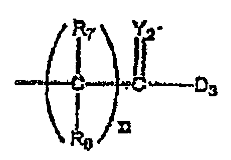 ist;
E₆₋₈ unabhängig voneinander H, E₅ oder 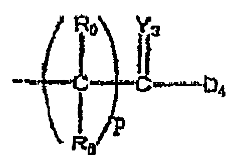 sind;
jedoch nicht alle Reste E₆₋₈ H sind;
D₃ und D₄ unabhängig voneinander OH, eine Abgangsgruppe, die mit einem ungeschützten Amin oder Hydroxy oder einer endständigen Verzweigungsgruppe wie in Anspruch 1 definiert reagieren kann, sind, jedoch nicht beide Reste D₃ und D₄ OH sind;
R₁ ein polymerer Rest ist;
Y₁ O, S oder NR₄ ist;
M O, S oder NR₅ ist;
(a) null oder eins ist;
(m) null oder eine positive ganze Zahl ist;
(n) und (p) unabhängig voneinander 0 oder eine positive ganze Zahl sind;
Y₂₋₃ unabhängig voneinander O, S oder NR₁₀ sind; und
R₂₋₁₀ unabhängig voneinander ausgewählt sind aus der Gruppe, bestehend aus Wasserstoff, C₁₋₆-Alkylen, verzweigten C₃₋₁₂-Alkylen, C₃₋₈-Cycloalkylen, substituierten C₁₋₆-Alkylen, substituierten C₃₋₈-Cycloalkylen, Arylen, substituierten Arylen, Aralkylen, C₁₋₆-Heteroalkylen, substituierten C₁₋₆-Heteroalkylen, C₁₋₆-Alkoxy, Phenoxy und C₁₋₆-Heteroalkoxy;
unter Bedingungen, die zum Bewirken der Bildung eines Polymerkonjugats ausreichend sind.

## Revendications

1. Composé de formule : 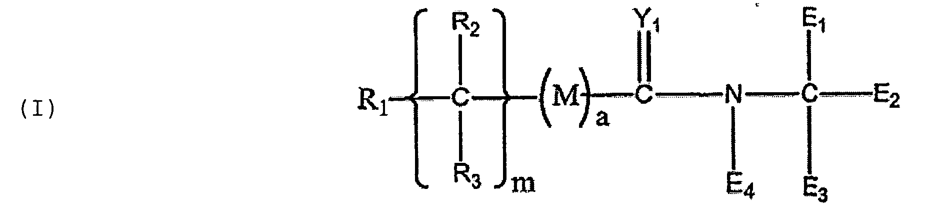 dans laquelle :
R₁ est un résidu polymère ;
Y₁ est O, S ou NR₄ ;
M est O, S ou NR₅ ;
E₁ est 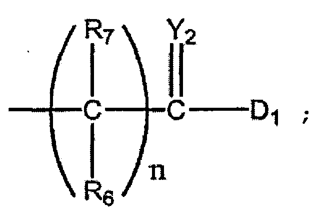
E₂ est E₁ ou 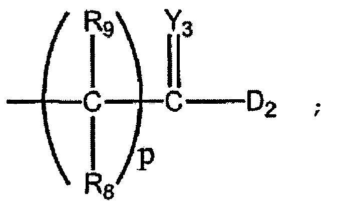
E₃ et E₄ sont indépendamment H, E₁ ou 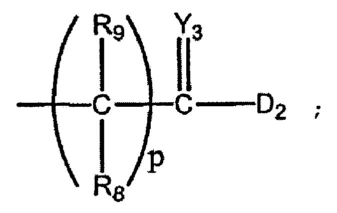
(a) vaut zéro ou un ;
(m) vaut zéro ou est un entier positif ;
(n) et (p) valent indépendamment zéro ou sont des entiers positifs ;
Y₂ et Y₃ sont indépendamment O, S ou NR₁₀ ;
R₂ à R₁₀ sont indépendamment choisis dans l'ensemble constitué par l'hydrogène et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆ ;
D₁ et D₂ sont indépendamment 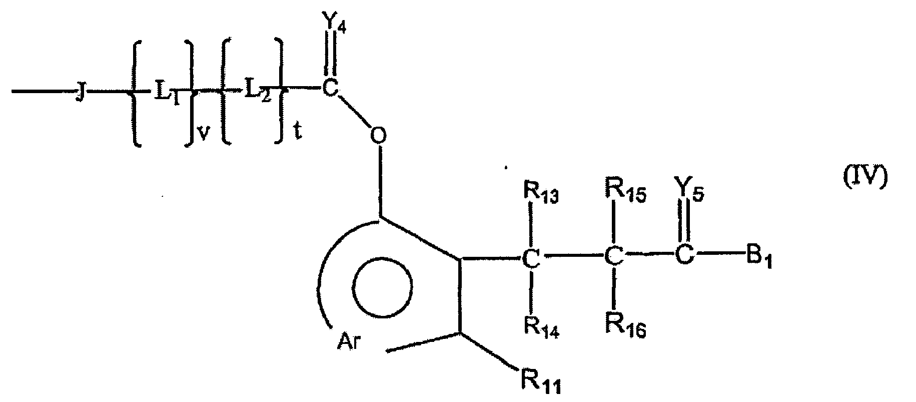 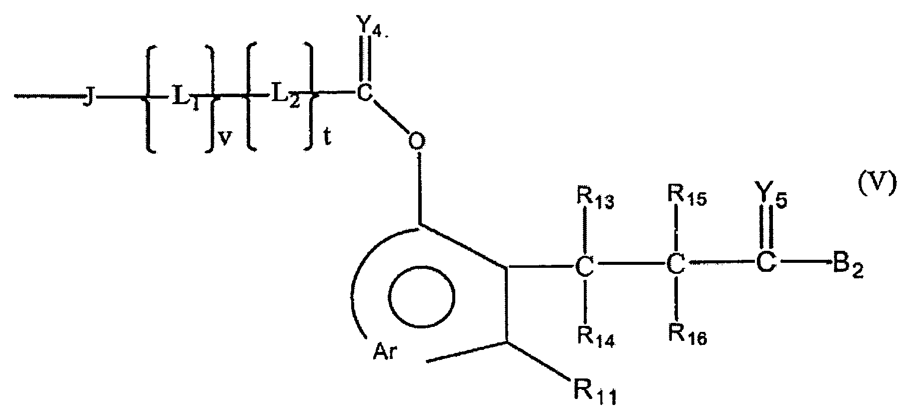
ou un groupe de ramification terminal ;
où (v) et (t) valent indépendamment 0 ou sont des entiers positifs allant jusqu'à environ 6 ;
J est NR₁₂ ou 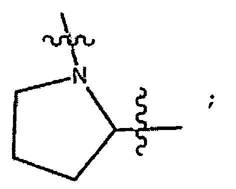
L₁ et L₂ sont indépendamment des lieurs bifonctionnels sélectionnés ;
Y₄ à Y₇ sont indépendamment choisis dans l'ensemble constitué par O, S et NR₁₄ ;
R₁₁ à R₁₄ sont indépendamment choisis dans l'ensemble constitué par l'hydrogène et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆ ;
Ar est un fragment qui, lorsqu'il est présent dans la formule (I), forme un groupe hydrocarboné aromatique multi-substitué ou un groupe hétérocyclique multi-substitué ;
B₁ et B₂ sont indépendamment choisis dans l'ensemble constitué par les groupes partants, OH, les résidus de fragments hydroxy-terminaux ou de fragments aminés ;
ledit groupe de ramification terminal étant de formule : 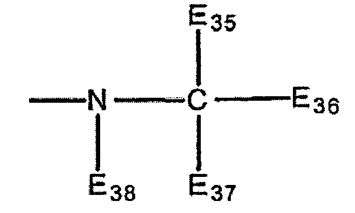
dans laquelle
E₃₅ est 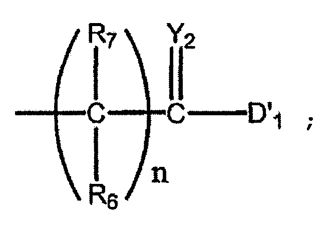
E₃₆ à E₃₈ sont indépendamment H, E₃₅ ou 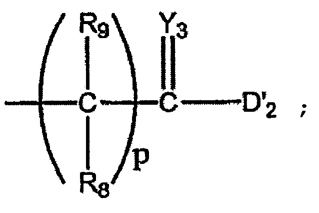
D'₁ est 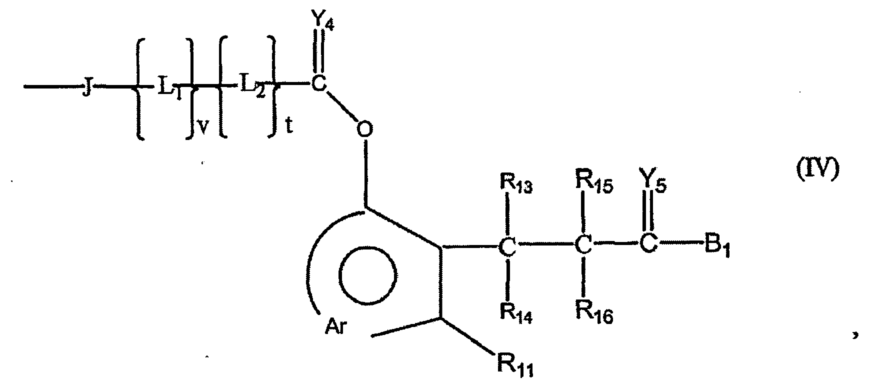 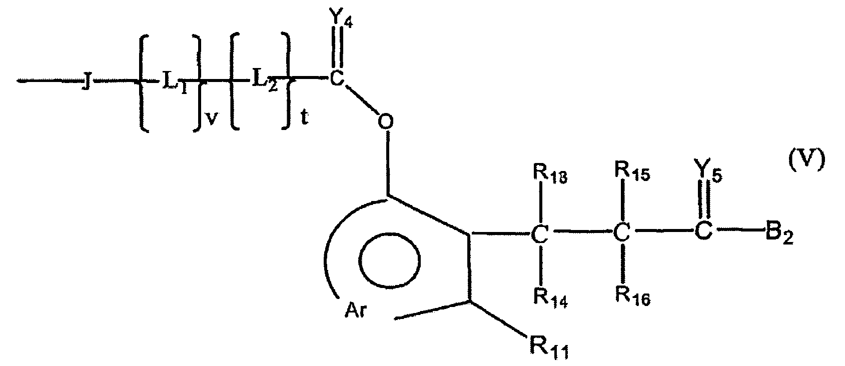 ou 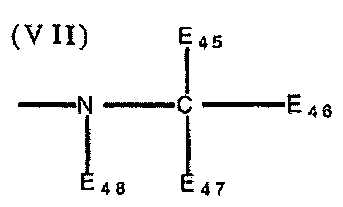
D'₂ est OH, 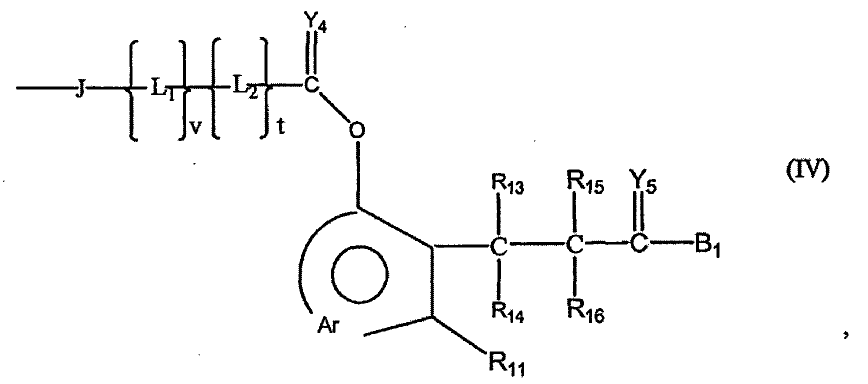 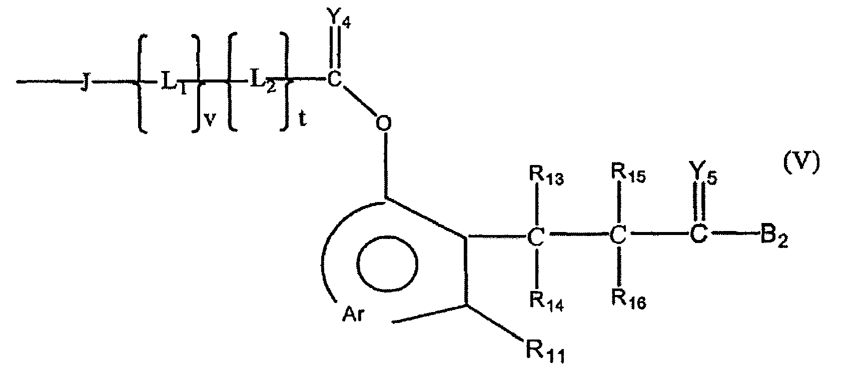 ou 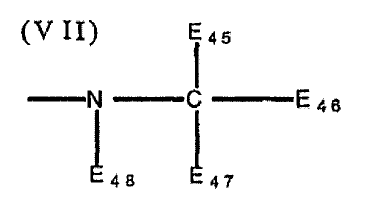 où
E₄₅ est 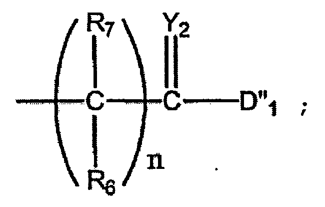
E₄₆ à E₄₈ sont indépendamment H, E₄₅ ou 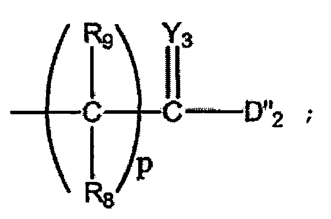
où D"₁ est 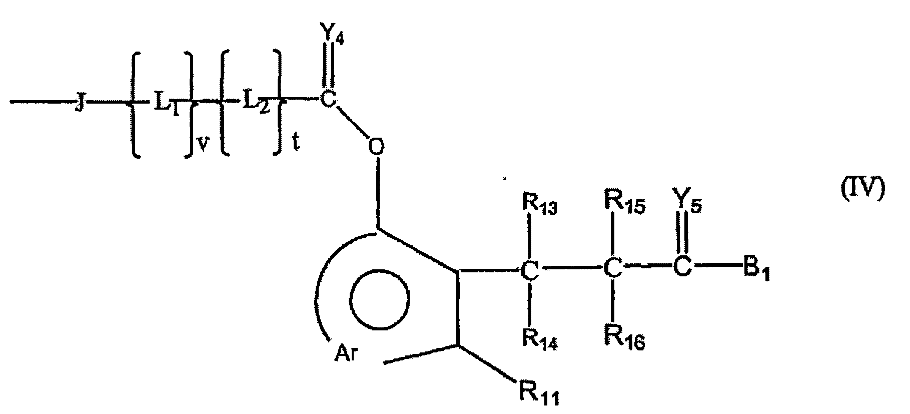 ou 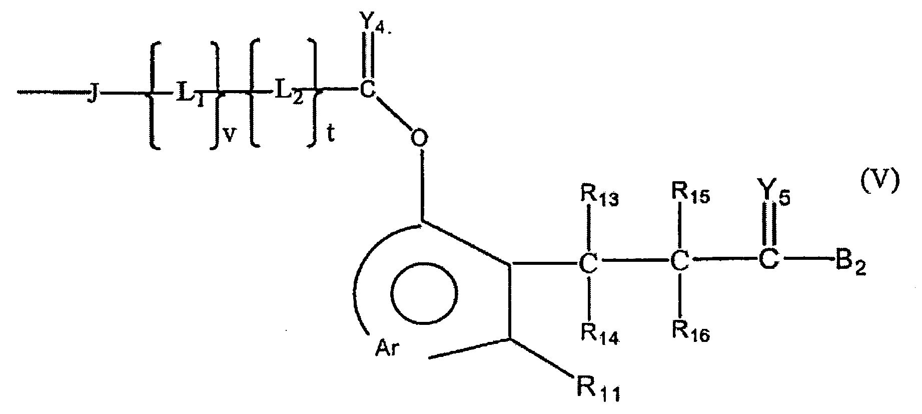
D"₂ est OH, 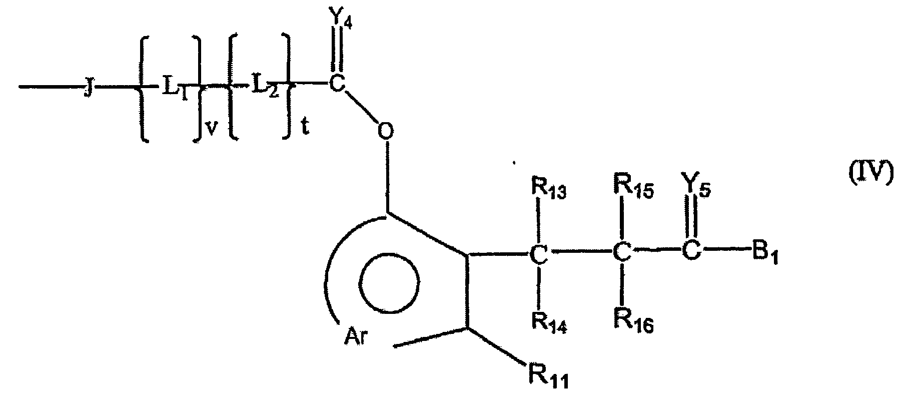 ou

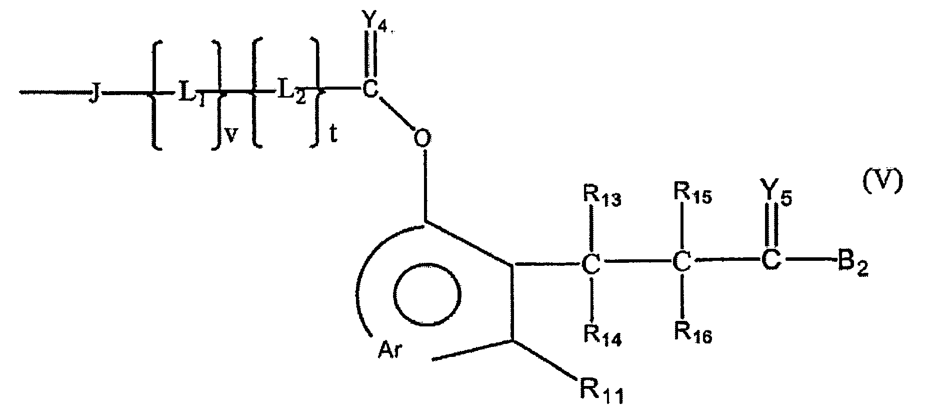

2. Composé selon la revendication 1, dans lequel R₁ comprend en outre un groupe de coiffage A choisi dans l'ensemble constitué par l'hydrogène, NH₂, OH, CO₂H, les fragments en C₁-C₆ et

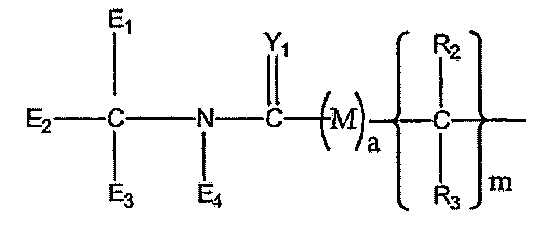

3. Composé selon la revendication 2, de formule :

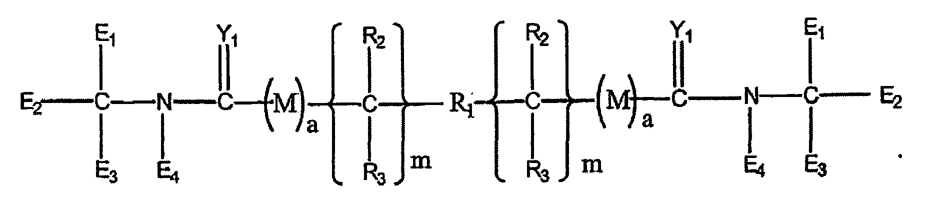

4. Composé selon la revendication 3, dans lequel Y₁ est O.

5. Composé selon la revendication 1, dans lequel R₁ comprend un résidu polyoxyalkylène.

6. Composé selon la revendication 5, dans lequel R₁ comprend un résidu polyéthylèneglycol.

7. Composé selon la revendication 3, dans lequel R₁ comprend un résidu polyéthylèneglycol.

8. Composé selon la revendication 5, dans lequel R₁ est choisi dans l'ensemble constitué par
-C(=Y₆)-(CH₂)_{f}-O-(CH₂CH₂O)ₓ-A,
-C(=Y₆)-Y₇-(CH₂)_{f}-O-(CH₂CH₂O)ₓ-A,
-C(=Y₆)-NR₂₃-(CH₂)_{f}-O-(CH₂CH₂O)ₓ-A,
-(CR₂₄R₂₅)ₑ-O-(CH₂)_{f}-O-(CH₂CH₂O)ₓ-A,
-NR₂₃-(CH₂)_{f}-O-(CH₂CH₂O)ₓ-A,
-C (=Y₆)-(CH₂)_{f}-O-(CH₂CH₂O)ₓ-(CH₂)_{f}-C(=Y₆)-,
-C (=Y₆)-Y₇-(CH₂)_{f}-O-(CH₂CH₂O)ₓ-(CH₂)_{f}-Y₇-C(=Y₆)-,
-C (₌Y₆₎-NR₂₃-(CH₂)_{f}-O-(CH₂CH₂O)ₓ-(CH₂)_{f}-NR₂₃-C(=Y₆)-,
- (CR₂₄R₂₅)ₑ-O-(CH₂)_{f}-O-(CH₂CH₂O)ₓ-(CH₂)_{f}-O-(CR₂₄R₂₅)ₑ-, et
-NMR₂₃-(CH₂)_{f}-O-(CH₂CH₂O)ₓ-(CH₂)_{f}-NR₂₃-
où : Y₆ et Y₇ sont indépendamment O, S ou NR₂₃ ;
x est le degré de polymérisation ;
R₂₃, R₂₄ et R₂₅ sont indépendamment choisis parmi H et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆ ;
e et f valent indépendamment zéro, un ou deux ; et
A est un groupe de coiffage.

9. Composé selon la revendication 8, dans lequel R₁ comprend -O-(CH₂CH₂O)ₓ et x est un entier positif tel que la masse moléculaire moyenne en masse soit d'au moins environ 20 000.

10. Composé selon la revendication 3, dans lequel R₁ a une masse moléculaire moyenne en masse d'environ 20 000 à environ 100 000.

11. Composé selon la revendication 3, dans lequel R₁ a une masse moléculaire moyenne en masse d'environ 25 000 à environ 60 000.

12. Composé selon la revendication 3, de formule :

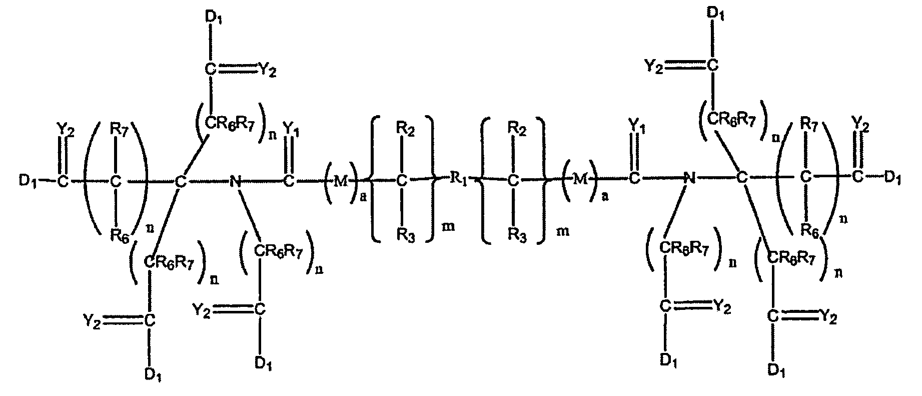

13. Composé selon la revendication 12, dans lequel D₁ est

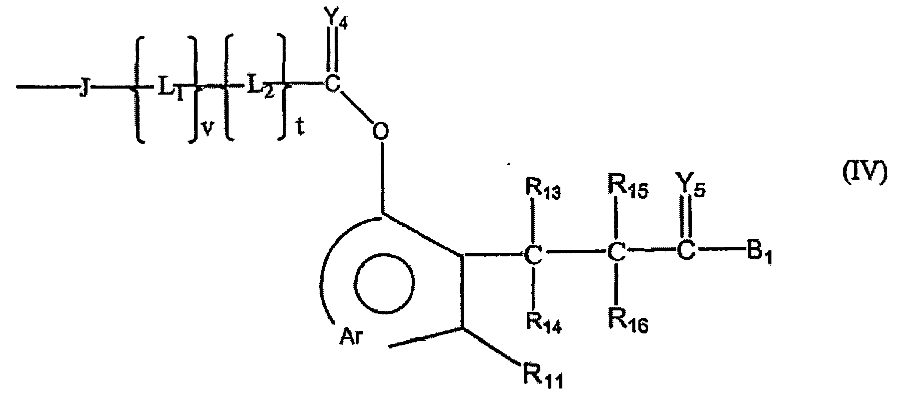

14. Composé selon la revendication 12, dans lequel D₁ est

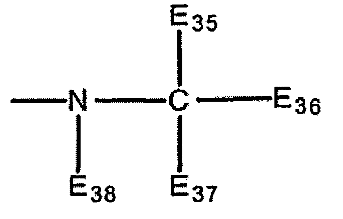

15. Composé selon la revendication 1, dans lequel L₁ est (CH₂CH₂O)₂.

16. Composé selon la revendication 1, dans lequel L₂ est choisi dans l'ensemble constitué par -CH₂-, -CH(CH₃₎-, -CH₂C(O)NHCH(CH₃)-, -(CH₂)₂-, -CH₂C(O)NHCH₂-, - (CH₂)₂-NH-, - (CH₂)₂-NH-C(O)(CH₂)₂NH et -CH₂C(O)NHCH (CH₂CH(CH₃)₂)-.

17. Composé selon la revendication 1, choisi dans l'ensemble constitué par 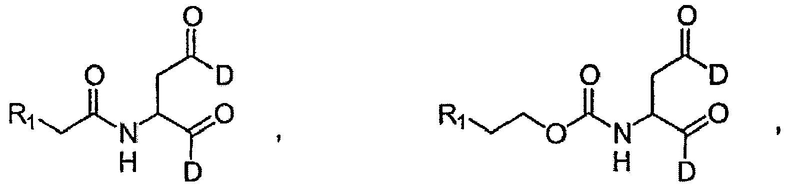 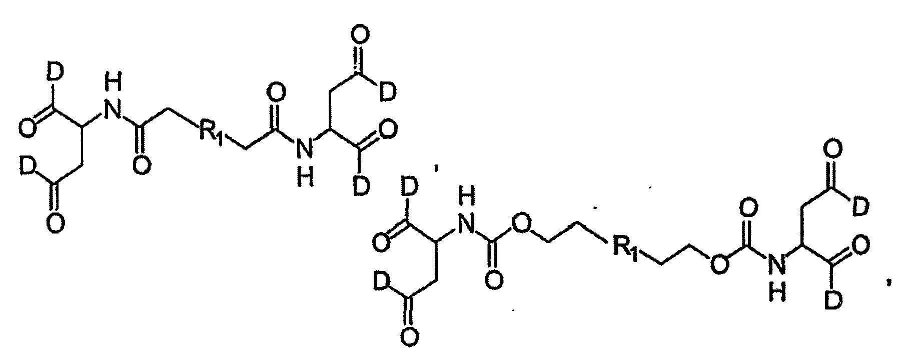 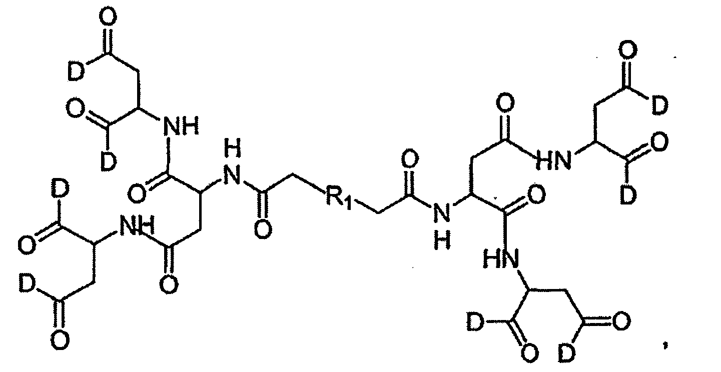 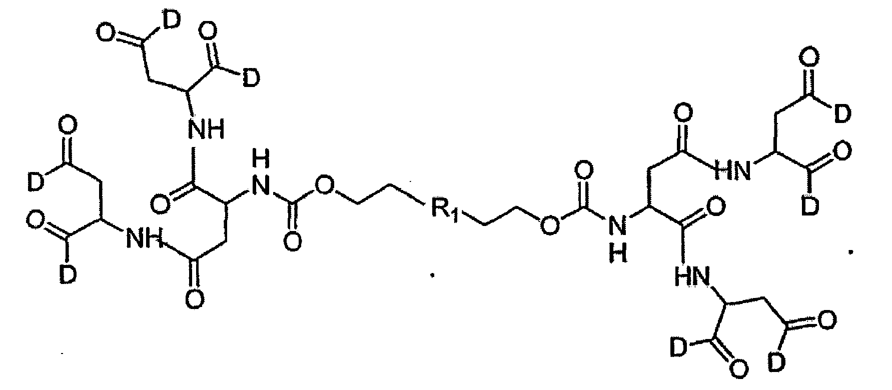 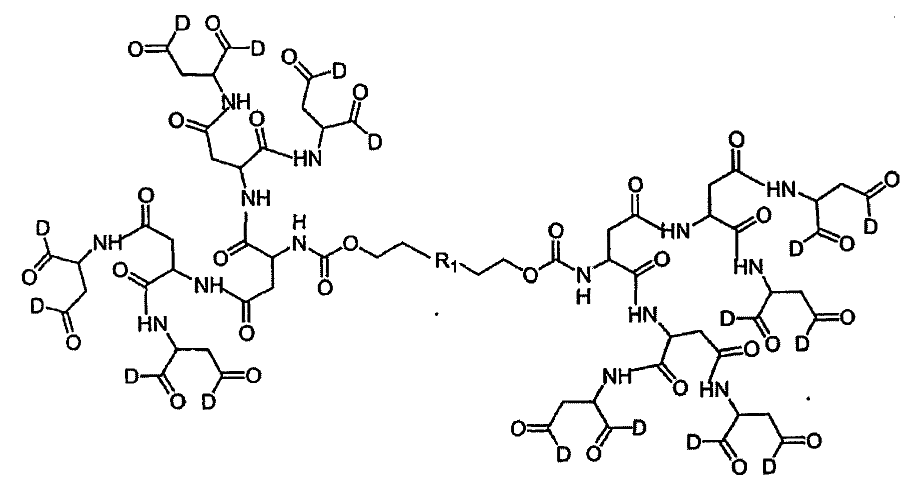 et 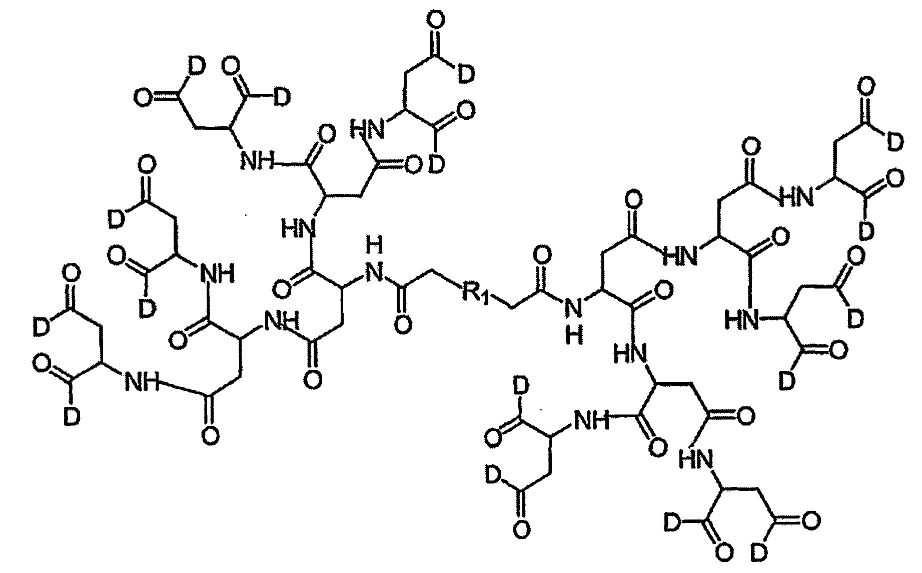 où R₁ est un résidu PEG et D est choisi dans l'ensemble constitué par : 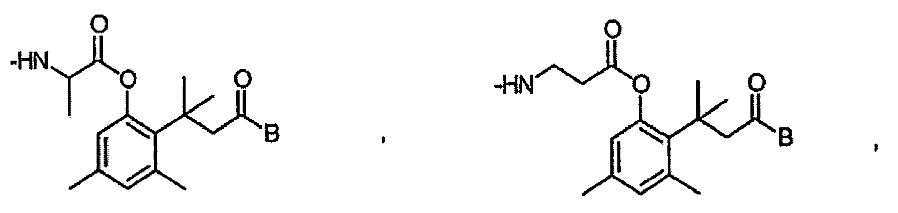 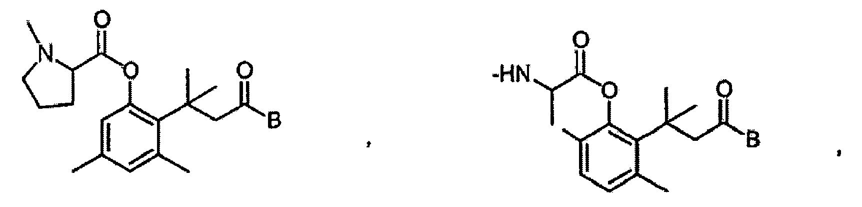 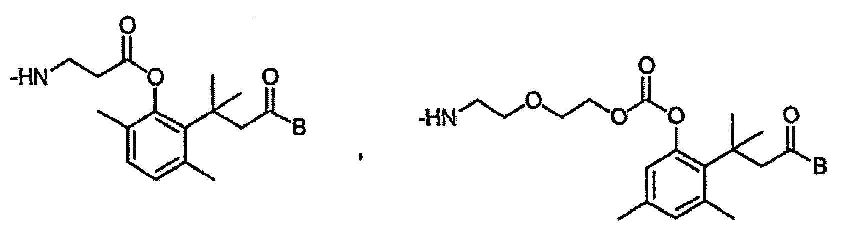 et 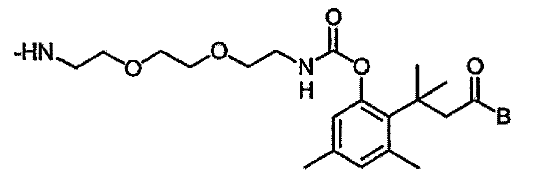 où B est un résidu d'une amine ou d'un médicament hydroxylé.

18. Composé selon la revendication 17, dans lequel B est un résidu d'un élément du groupe constitué par : la daunorubicine, la doxorubicine ; la moutarde de p-aminoaniline, le malphalan, l'Ara-C (cytosine arabinoside), la leucine-Ara-C, et la gemcitabine.

19. Composition pharmaceutique comprenant un composé pro-médicament tel que défini dans l'une quelconque des revendications 1 à 18 et un véhicule acceptable en pharmacie.

20. Composé tel que défini dans l'une quelconque des revendications 1 à 18, pour une utilisation dans une méthode de traitement du corps humain ou animal par thérapie.

21. Composé selon la revendication 1, dans lequel Ar est de formule : 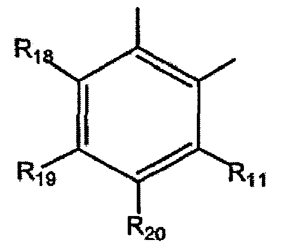 dans laquelle R₁₁ et R₁₈ à R₂₀ sont individuellement choisis dans l'ensemble constitué par l'hydrogène et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆.

22. Composé selon la revendication 21, dans lequel chacun de R₁₁ et R₁₈ à R₂₀ est H ou CH₃.

23. Procédé pour préparer un conjugué de polymère, comprenant :
la réaction d'un composé de formule (VIII) : 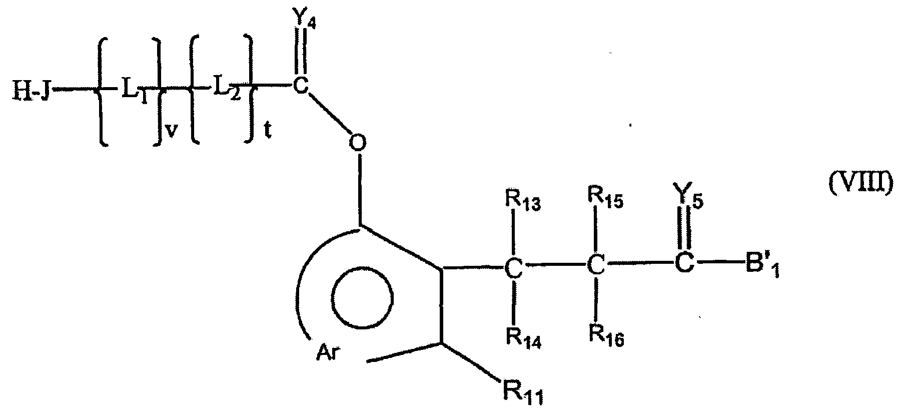
dans laquelle
(v) et (t) valent indépendamment 0 ou sont des entiers positifs allant jusqu'à environ 6 ;
J est NR₁₂ ou 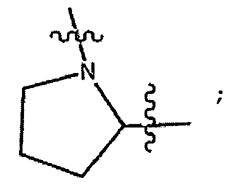
L₁ et L₂ sont indépendamment des lieurs bifonctionnels sélectionnés ;
Y₄ et Y₅ sont indépendamment choisis dans l'ensemble constitué par O, S et NR₁₇ ;
R₁₁ à R₁₇ sont indépendamment choisis dans l'ensemble constitué par l'hydrogène et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆ ;
Ar est un fragment qui, lorsqu'il est présent dans la formule (I), forme un groupe hydrocarboné aromatique multi-substitué ou un groupe hétérocyclique multi-substitué ; et
B'₁ est un résidu d'un fragment hydroxylé ou aminé ;
avec un composé de formule (IX) : 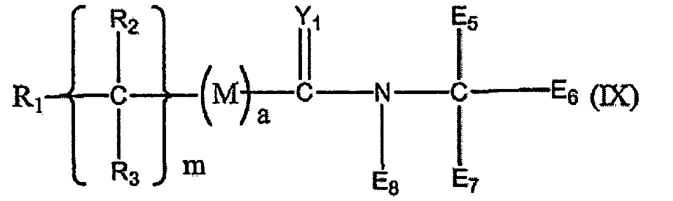
dans laquelle
E₅ est 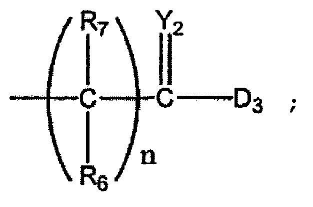
E₆ à E₈ sont indépendamment H, E₅ ou 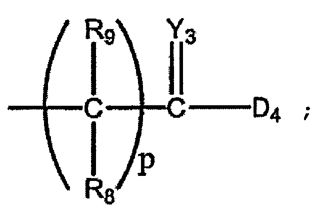
mais E₆ à E₈ ne sont pas tous H ;
D₃ et D₄ sont indépendamment OH, un groupe partant qui est capable de réagir avec un radical amine ou hydroxyle non protégé ou un groupe de ramification terminal tel que défini dans la revendication 1, mais D₃ et D₄ ne sont pas tous OH ;
R₁ est un résidu polymère ;
Y₁ est O, S ou NR₄ ;
M est O, S ou NR₅ ;
(a) vaut zéro ou un ;
(m) vaut 0 ou est un entier positif ;
(n) et (p) valent indépendamment 0 ou sont des entiers positifs ;
Y₂ et Y₃ sont indépendamment O, S ou NR₁₀ ; et
R₂ à R₁₀ sont indépendamment choisis dans l'ensemble constitué par l'hydrogène et les radicaux alkyle en C₁-C₆, alkyle ramifiés en C₃-C₁₂, cycloalkyle en C₃-C₈, alkyle substitués en C₁-C₆, cycloalkyle substitués en C₃-C₈, aryle, aryle substitués, aralkyle, hétéroalkyle en C₁-C₆, hétéroalkyle en C₁-C₆ substitués, alcoxy en C₁-C₆, phénoxy et hétéroalcoxy en C₁-C₆ ;
dans des conditions suffisantes pour provoquer la formation d'un conjugué polymère.
